# EUROPEAN PATENT SPECIFICATION

(11) **EP 2 222 848 B1**
(45) Date of publication and mention of the grant of the patent: **25.02.2015**
(21) Application number: 08861334.4
(22) Date of filing: 18.12.2008
(51) Int. Cl.: C12N 15/113

(54) **NOVEL SIRNA STRUCTURE FOR MINIMIZING OFF-TARGET EFFECTS AND RELAXING SATURATION OF RNAI MACHINERY AND THE USE THEREOF**
NEUE SIRNA-STRUKTUR ZUR MINIMIERUNG VON OFF-TARGET-EFFEKTEN UND RELAXATION DER SÄTTIGUNG DER RNAI-MASCHINERIE SOWIE VERWENDUNG DAVON
NOUVELLE STRUCTURE DE PETIT ARN INTERFÉRENT PERMETTANT D'ATTÉNUER LES EFFETS HORS CIBLE ET D'ALLÉGER LA SATURATION DE MÉCANISME D'ARN INTERFÉRENT, ET UTILISATION

(30) Priority: 18.12.2007 KR 20070133416
(43) Date of publication of application: 01.09.2010
(73) Proprietor: Lee, Dong Ki, Seoul 137-775 (KR)
(72) Inventor: CHANG, Chan Il, Daejeon 302-241 (KR)
(74) Representative: Hiebl, Inge Elisabeth
(86) International application number: PCT/KR2008/007530
(87) International publication number: WO 2009/078685

(56) References cited:
- WO-A2-02/055693
- WO-A2-2004/080406
- WO-A2-2005/062937
- WO-A2-2005/079533
- WO-A2-2009/029688
- US-A1- 2004 180 351
- US-A1- 2006 142 228
- US-A1- 2006 160 123
- US-A1- 2007 218 495
- US-B2- 7 078 196
- BRAMSEN JESPER B ET AL: "Improved silencing properties using small internally segmented interfering RNAs", NUCLEIC ACIDS RESEARCH, OXFORD UNIVERSITY PRESS, SURREY, GB, vol. 35, no. 17, 1 January 2007 (2007-01-01), pages 5886-5897, XP002458184, ISSN: 0305-1048, DOI: DOI:10.1093/NAR/GKM548
- ROSE S D ET AL: "Functional polarity is introduced by Dicer processing of short substrate RNAs", NUCLEIC ACIDS RESEARCH, OXFORD UNIVERSITY PRESS, SURREY, GB, vol. 33, no. 13, 26 July 2005 (2005-07-26) , pages 4140-4156, XP002399886, ISSN: 0305-1048, DOI: DOI:10.1093/NAR/GKI732
- SOUTSCHEK JUERGEN ET AL: "Therapeutic silencing of an endogenous gene by systemic administration of modified siRNAs", NATURE, NATURE PUBLISHING GROUP, LONDON, GB, vol. 432, no. 7014, 11 November 2004 (2004-11-11), pages 173-178, XP002333747, ISSN: 0028-0836, DOI: DOI:10.1038/NATURE03121
- CHANG CHAN IL ET AL: "Asymmetric shorter-duplex siRNA structures trigger efficient gene silencing with reduced nonspecific effects.", MOLECULAR THERAPY : THE JOURNAL OF THE AMERICAN SOCIETY OF GENE THERAPY APR 2009 LNKD- PUBMED:19156133, vol. 17, no. 4, April 2009 (2009-04), pages 725-732, XP002635991, ISSN: 1525-0024
- KUMIKO UI-TEI ET AL.: 'Essential Notes Regarding the Design of Functional siRNAs for Efficient Mammalian RNAi' J. BIOMED. BIOTECHNOL. vol. 2006, no. 65052, 2006, pages 1 - 8
- SUN XIANGAO ET AL: "Asymmetric RNA duplexes mediate RNA interference in mammalian cells", NATURE BIOTECHNOLOGY, NATURE PUBLISHING GROUP, NEW YORK, NY, US, vol. 26, no. 12, 1 December 2008 (2008-12-01), pages 1379-1382, XP002639662, ISSN: 1087-0156, DOI: 10.1038/NBT.1512 [retrieved on 2008-11-23]

<110> LEE, Dong Ki
<120> Novel siRNA Structure for Minimizing Off-target Effects and Relaxing Saturation of RNAi Machinery and the Use Thereof
<130> PP-B615
<150> KR10-2007-0133416
   <151> 2007-12-18
<160> 93
<170> KopatentIn 1.71
<210> 1
   <211> 21
   <212> DNA/RNA
   <213> Artificial Sequence
<220>
   <223> antisense strand of siRNA for TIG3 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 1
   uagagaacgc cugagacagt t 21
<210> 2
   <211> 21
   <212> DNA/RNA
   <213> Artificial Sequence
<220>
   <223> sense strand of siRNA for TIG3 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 2
   cugucucagg cguucucuat t 21
<210> 3
   <211> 19
   <212> RNA
   <213> Artificial Sequence
<220>
   <223> antisense strand of siRNA for TIG3 mRNA
<400> 3
   uagagaacgc cugagacag 19
<210> 4
   <211> 19
   <212> RNA
   <213> Artificial Sequence
<220>
   <223> sense strand of siRNA for TIG3 mRNA
<400> 4
   cugucucagg cguucucua 19
<210> 5
   <211> 17
   <212> RNA
   <213> Artificial Sequence
<220>
   <223> antisense strand of siRNA for TIG3 mRNA
<400> 5
   uagagaacgc cugagac 17
<210> 6
   <211> 17
   <212> RNA
   <213> Artificial Sequence
<220>
   <223> sense strand of siRNA for TIG3 mRNA
<400> 6
   gucucaggcg uucucua 17
<210> 7
   <211> 16
   <212> RNA
   <213> Artificial Sequence
<220>
   <223> antisense strand of siRNA for TIG3 mRNA
<400> 7
   uagagaacgc cugaga 16
<210> 8
   <211> 16
   <212> RNA
   <213> Artificial Sequence
<220>
   <223> sense strand of siRNA for TIG3 mRNA
<400> 8
   ucucaggcgu ucucua 16
<210> 9
   <211> 15
   <212> RNA
   <213> Artificial Sequence
<220>
   <223> antisense strand of siRNA for TIG3 mRNA
<400> 9
   uagagaacgc cugag 15
<210> 10
   <211> 15
   <212> RNA
   <213> Artificial Sequence
<220>
   <223> sense strand of siRNA for TIG3 mRNA
<400> 10
   cucaggcguu cucua 15
<210> 11
   <211> 13
   <212> RNA
   <213> Artificial Sequence
<220>
   <223> antisense strand of siRNA for TIG3 mRNA
<400> 11
   uagagaacgc cug 13
<210> 12
   <211> 13
   <212> RNA
   <213> Artificial Sequence
<220>
   <223> sense strand of siRNA for TIG3 mRNA
<400> 12
   caggcguucu cua 13
<210> 13
   <211> 20
   <212> DNA
   <213> Artificial Sequence
<220>
   <223> TIG3-forward primer
<400> 13
   agattttccg ccttggctat 20
<210> 14
   <211> 20
   <212> DNA
   <213> Artificial Sequence
<220>
   <223> TIG3-reverse primer
<400> 14
   tttcacctct gcactgttgc 20
<210> 15
   <211> 21
   <212> DNA/RNA
   <213> Artificial Sequence
<220>
   <223> antisense strand of siRNA for LaminA/C mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 15
   uguucuucug gaaguccagt t 21
<210> 16
   <211> 21
   <212> DNA/RNA
   <213> Artificial Sequence
<220>
   <223> sense strand of siRNA for LaminA/C mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 16
   cuggacuucc agaagaacat t 21
<210> 17
   <211> 19
   <212> RNA
   <213> Artificial Sequence
<220>
   <223> antisense strand of siRNA for LaminA/C mRNA
<400> 17
   uguucuucug gaaguccag 19
<210> 18
   <211> 19
   <212> RNA
   <213> Artificial Sequence
<220>
   <223> sense strand of siRNA for LaminA/C mRNA
<400> 18
   cuggacuucc agaagaaca 19
<210> 19
   <211> 17
   <212> RNA
   <213> Artificial Sequence
<220>
   <223> antisense strand of siRNA for LaminA/C mRNA
<400> 19
   uguucuucug gaagucc 17
<210> 20
   <211> 17
   <212> RNA
   <213> Artificial Sequence
<220>
   <223> sense strand of siRNA for LaminA/C mRNA
<400> 20
   ggacuuccag aagaaca 17
<210> 21
   <211> 15
   <212> RNA
   <213> Artificial Sequence
<220>
   <223> sense strand of siRNA for LaminA/C mRNA
<400> 21
   acuuccagaa gaaca 15
<210> 22
   <211> 21
   <212> DNA/RNA
   <213> Artificial Sequence
<220>
   <223> antisense strand of siRNA for Survivin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 22
   ugaaaauguu gaucuccuut t 21
<210> 23
   <211> 21
   <212> DNA/RNA
   <213> Artificial Sequence
<220>
   <223> sense strand of siRNA for Survivin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 23
   aaggagauca acauuuucat t 21
<210> 24
   <211> 19
   <212> RNA
   <213> Artificial Sequence
<220>
   <223> antisense strand of siRNA for Survivin mRNA
<400> 24
   ugaaaauguu gaucuccuu 19
<210> 25
   <211> 19
   <212> RNA
   <213> Artificial Sequence
<220>
   <223> sense strand of siRNA for Survivin mRNA
<400> 25
   aaggagauca acauuuuca 19
<210> 26
   <211> 17
   <212> RNA
   <213> Artificial Sequence
<220>
   <223> antisense strand of siRNA for Survivin mRNA
<400> 26
   ugaaaauguu gaucucc 17
<210> 27
   <211> 17
   <212> RNA
   <213> Artificial Sequence
<220>
   <223> sense strand of siRNA for Survivin mRNA
<400> 27
   ggagaucaac auuuuca 17
<210> 28
   <211> 15
   <212> RNA
   <213> Artificial Sequence
<220>
   <223> sense strand of siRNA for Survivin mRNA
<400> 28
   agaucaacau uuuca 15
<210> 29
   <211> 20
   <212> DNA
   <213> Artificial Sequence
<220>
   <223> Lamin-forward primer
<400> 29
   ccgagtctga agaggtggtc 20
<210> 30
   <211> 20
   <212> DNA
   <213> Artificial Sequence
<220>
   <223> Lamin-reverse primer
<400> 30
   aggtcaccct ccttcttggt 20
<210> 31
   <211> 20
   <212> DNA
   <213> Artificial Sequence
<220>
   <223> Survivin-forward primer
<400> 31
   gcaccacttc cagggtttat 20
<210> 32
   <211> 20
   <212> DNA
   <213> Artificial Sequence
<220>
   <223> Survivin-reverse primer
<400> 32
   ctctggtgcc actttcaaga 20
<210> 33
   <211> 16
   <212> RNA
   <213> Artificial Sequence
<220>
   <223> sense strand of siRNA for LaminA/C mRNA
<400> 33
   gacuuccaga agaaca 16
<210> 34
   <211> 16
   <212> RNA
   <213> Artificial Sequence
<220>
   <223> sense strand of siRNA for Survivin mRNA
<400> 34
   gagaucaaca uuuuca 16
<210> 35
   <211> 14
   <212> RNA
   <213> Artificial Sequence
<220>
   <223> sense strand of siRNA for TIG3 mRNA
<400> 35
   ucaggcguuc ucua 14
<210> 36
   <211> 15
   <212> RNA
   <213> Artificial Sequence
<220>
   <223> antisense strand of siRNA for TIG3 mRNA
<400> 36
   gaacgccuga gacag 15
<210> 37
   <211> 17
   <212> RNA
   <213> Artificial Sequence
<220>
   <223> antisense strand of siRNA for LaminA/C mRNA
<400> 37
   uucuucugga aguccag 17
<210> 38
   <211> 17
   <212> RNA
   <213> Artificial Sequence
<220>
   <223> sense strand of siRNA for LaminA/C mRNA
<400> 38
   cuggacuucc agaagaa 17
<210> 39
   <211> 15
   <212> RNA
   <213> Artificial Sequence
<220>
   <223> sense strand of siRNA for TIG3 mRNA
<400> 39
   cugucucagg cguuc 15
<210> 40
   <211> 21
   <212> DNA/RNA
   <213> Artificial Sequence
<220>
   <223> antisense strand of siRNA for Integrin mRNA
<220>
   <223> Molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 40
   auaucugaag ugcaguucat t 21
<210> 41
   <211> 21
   <212> DNA/RNA
   <213> Artificial Sequence
<220>
   <223> sense strand of siRNA for Integrin mRNA
<220>
   <223> Molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 41
   ugaacugcac uucagauaut t 21
<210> 42
   <211> 19
   <212> RNA
   <213> Artificial Sequence
<220>
   <223> antisense strand of siRNA for Integrin mRNA
<400> 42
   auaucugaag ugcaguuca 19
<210> 43
   <211> 17
   <212> RNA
   <213> Artificial Sequence
<220>
   <223> sense strand of siRNA for Integrin mRNA
<400> 43
   aacugcacuu cagauau 17
<210> 44
   <211> 16
   <212> RNA
   <213> Artificial Sequence
<220>
   <223> sense strand of siRNA for Integrin mRNA
<400> 44
   acugcacuuc agauau 16
<210> 45
   <211> 20
   <212> DNA
   <213> Artificial Sequence
<220>
   <223> Integrin-forward primer
<400> 45
   cgtatctgcg ggatgaatct 20
<210> 46
   <211> 20
   <212> DNA
   <213> Artificial Sequence
<220>
   <223> Integrin-reverse primer
<400> 46
   gggttgcaag cctgttgtat 20
<210> 47
   <211> 20
   <212> DNA
   <213> Artificial Sequence
<220>
   <223> Calcineurin-forward primer
<400> 47
   gcaaccatga atgcagacac 20
<210> 48
   <211> 20
   <212> DNA
   <213> Artificial Sequence
<220>
   <223> Calcineurin-reverse primer
<400> 48
   tggtgaaagt ccaccatgaa 20
<210> 49
   <211> 20
   <212> DNA
   <213> Artificial Sequence
<220>
   <223> ATF6-forward primer
<400> 49
   gcctttattg cttccagcag 20
<210> 50
   <211> 20
   <212> DNA
   <213> Artificial Sequence
<220>
   <223> ATF6-reverse primer
<400> 50
   tgagacagca aaaccgtctg 20
<210> 51
   <211> 20
   <212> DNA
   <213> Artificial Sequence
<220>
   <223> DBP-forward primer
<400> 51
   gtagacctgg acgccttcct 20
<210> 52
   <211> 20
   <212> DNA
   <213> Artificial Sequence
<220>
   <223> DBP-reverse primer
<400> 52
   cgggttcaaa ggtcatcaac 20
<210> 53
   <211> 20
   <212> DNA
   <213> Artificial Sequence
<220>
   <223> TEF-forward primer
<400> 53
   ccccagccta tgatcaaaaa 20
<210> 54
   <211> 20
   <212> DNA
   <213> Artificial Sequence
<220>
   <223> TEF-reverse primer
<400> 54
   ccggatggtg atctgattct 20
<210> 55
   <211> 20
   <212> DNA
   <213> Artificial Sequence
<220>
   <223> HIF1alpha-forward primer
<400> 55
   ccagcaacag aaagtcgtca 20
<210> 56
   <211> 20
   <212> DNA
   <213> Artificial Sequence
<220>
   <223> HIF1alpha-reverse primer
<400> 56
   ggctatactt gggcatggaa 20
<210> 57
   <211> 20
   <212> DNA
   <213> Artificial Sequence
<220>
   <223> NF-kB-forward primer
<400> 57
   cctggagcag gctatcagtc 20
<210> 58
   <211> 20
   <212> DNA
   <213> Artificial Sequence
<220>
   <223> NF-kB-reverse primer
<400> 58
   cactgtcacc tggaagcaga 20
<210> 59
   <211> 25
   <212> DNA
   <213> Artificial Sequence
<220>
   <223> TIG3 Gene specific 3' primer
<400> 59
   ggggcagatg gctgtttatt gatcc 25
<210> 60
   <211> 25
   <212> DNA
   <213> Artificial Sequence
<220>
   <223> TIG3 Gene specific 3' nested primer
<400> 60
   acttttgcca gcgagagagg gaaac 25
<210> 61
   <211> 25
   <212> DNA
   <213> Artificial Sequence
<220>
   <223> Lamin Gene specific 3' primer
<400> 61
   ccagtgagtc ctccaggtct cgaag 25
<210> 62
   <211> 24
   <212> DNA
   <213> Artificial Sequence
<220>
   <223> Lamin Gene specific 3' nested primer
<400> 62
   cctggcattg tccagcttgg caga 24
<210> 63
   <211> 21
   <212> DNA/RNA
   <213> Artificial Sequence
<220>
   <223> antisense strand of siRNA for CREB3 mRNA
<220>
   <223> Molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 63
   ggcucagacu guguacucct t 21
<210> 64
   <211> 21
   <212> DNA/RNA
   <213> Artificial Sequence
<220>
   <223> sense strand of siRNA for CREB3 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotidea
<400> 64
   ggaguacaca gucugagcct t 21
<210> 65
   <211> 19
   <212> DNA
   <213> Artificial Sequence
<220>
   <223> DNA fragment that encodes sense-target of siSurvivin
<400> 65
   tgaaaatgtt gatctcctt 19
<210> 66
   <211> 19
   <212> DNA
   <213> Artificial Sequence
<220>
   <223> DNA fragment that encodes antisense-target of siSurvivin
<400> 66
   aaggagatca acattttca 19
<210> 67
   <211> 19
   <212> RNA
   <213> Artificial Sequence
<220>
   <223> complementary fragment for siSurvivin sense strand
<400> 67
   ugaaaauguu gaucuccuu 19
<210> 68
   <211> 19
   <212> RNA
   <213> Artificial Sequence
<220>
   <223> complementary fragment for siSurvivin antisense strand
<400> 68
   aaggagauca acauuuuca 19
<210> 69
   <211> 19
   <212> RNA
   <213> Artificial Sequence
<220>
   <223> sense strand of siRNA for Survivin mRNA
<400> 69
   gagaucaaca uuuucaaau 19
<210> 70
   <211> 19
   <212> RNA
   <213> Artificial Sequence
<220>
   <223> sense strand of siRNA for TIG3 mRNA
<400> 70
   ucucaggcgu ucucuagau 19
<210> 71
   <211> 21
   <212> DNA/RNA
   <213> Artificial Sequence
<220>
   <223> sense strand of siRNA for Calcineurin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 71
   gugaugaaua uucgacagut t 21
<210> 72
   <211> 21
   <212> DNA/RNA
   <213> Artificial Sequence
<220>
   <223> antisense strand of siRNA for Calcineurin mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 72
   acugucgaau auucaucact t 21
<210> 73
   <211> 16
   <212> RNA
   <213> Artificial Sequence
<220>
   <223> sense strand of siRNA for Calcineurin mRNA
<400> 73
   augaauauuc gacagu 16
<210> 74
   <211> 21
   <212> DNA/RNA
   <213> Artificial Sequence
<220>
   <223> sense strand of siRNA for ATF6 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 74
   ccagccuccu caaguuauut t 21
<210> 75
   <211> 21
   <212> DNA/RNA
   <213> Artificial Sequence
<220>
   <223> antisense strand of siRNA for ATF6 mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 75
   aauaacuuga ggaggcuggt t 21
<210> 76
   <211> 16
   <212> RNA
   <213> Artificial Sequence
<220>
   <223> sense strand of siRNA for ATF6 mRNA
<400> 76
   gccuccucaa guuauu 16
<210> 77
   <211> 21
   <212> DNA/RNA
   <213> Artificial Sequence
<220>
   <223> sense strand of siRNA for DBP mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 77
   ucgaagacau cgcuucucat t 21
<210> 78
   <211> 21
   <212> DNA/RNA
   <213> Artificial Sequence
<220>
   <223> antisense strand of siRNA for DBP mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 78
   ugagaagcga ugucuucgat t 21
<210> 79
   <211> 16
   <212> RNA
   <213> Artificial Sequence
<220>
   <223> sense strand of siRNA for DBP mRNA
<400> 79
   aagacaucgc uucuca 16
<210> 80
   <211> 21
   <212> DNA/RNA
   <213> Artificial Sequence
<220>
   <223> sense strand of siRNA for TEF mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 80
   caagacgcaa gaagaacaat t 21
<210> 81
   <211> 21
   <212> DNA/RNA
   <213> Artificial Sequence
<220>
   <223> antisense strand of siRNA for TEF mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 81
   uuguucuucu ugcgucuugt t 21
<210> 82
   <211> 16
   <212> RNA
   <213> Artificial Sequence
<220>
   <223> sense strand of siRNA for TEF mRNA
<400> 82
   gacgcaagaa gaacaa 16
<210> 83
   <211> 21
   <212> DNA/RNA
   <213> Artificial Sequence
<220>
   <223> sense strand of siRNA for HIF-1alpha mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 83
   ccuacugcag ggugaagaat t 21
<210> 84
   <211> 21
   <212> DNA/RNA
   <213> Artificial Sequence
<220>
   <223> antisense strand of siRNA for HIF-1alpha mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 84
   uucuucaccc ugcaguaggt t 21
<210> 85
   <211> 16
   <212> RNA
   <213> Artificial Sequence
<220>
   <223> sense strand of siRNA for HIF-1alpha mRNA
<400> 85
   acugcagggu gaagaa 16
<210> 86
   <211> 21
   <212> DNA/RNA
   <213> Artificial Sequence
<220>
   <223> sense strand of siRNA for HIF-1alpha mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 86
   ggguaaagaa caaaacacat t 21
<210> 87
   <211> 21
   <212> DNA/RNA
   <213> Artificial Sequence
<220>
   <223> antisense strand of siRNA for HIF-1alpha mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 87
   uguguuuugu ucuuuaccct t 21
<210> 88
   <211> 16
   <212> RNA
   <213> Artificial Sequence
<220>
   <223> sense strand of siRNA for HIF-1alpha mRNA
<400> 88
   uaaagaacaa aacaca 16
<210> 89
   <211> 21
   <212> DNA/RNA
   <213> Artificial Sequence
<220>
   <223> sense strand of siRNA for NF-kB mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 89
   gcccuauccc uuuacgucat t 21
<210> 90
   <211> 21
   <212> DNA/RNA
   <213> Artificial Sequence
<220>
   <223> antisense strand of siRNA for NF-kB mRNA
<220>
   <223> molecule is combined RNA/DNA
<220>
   <221> misc_feature
   <222> (1)..(19)
   <223> ribonucleotides
<220>
   <221> misc_feature
   <222> (20)..(21)
   <223> deoxyribonucleotides
<400> 90
   ugacguaaag ggauagggct t 21
<210> 91
   <211> 16
   <212> RNA
   <213> Artificial Sequence
<220>
   <223> sense strand of siRNA for NF-kB mRNA
<400> 91
   cuaucccuuu acguca 16
<210> 92
   <211> 36
   <212> RNA
   <213> Artificial Sequence
<220>
   <223> mRNA sequence of TIG3
<400> 92
   ugcccugucu caggcguucu cuagauccuu uccucu 36
<210> 93
   <211> 36
   <212> RNA
   <213> Artificial Sequence
<220>
   <223> mRNA sequence of LaminA/C
<400> 93
   ggaacuggac uuccagaaga acaucuacag ugagga 36

## Description

### TECHNICAL FIELD

The present invention relates to a novel siRNA structure and the use thereof, and more particularly to a novel siRNA molecule, which has high gene silencing efficiency, does not saturate the RNAi machinery and minimizes off-target effects caused by the siRNA sense strand.

### BACKGROUND ART

RNA interference (hereinafter abbreviated as "RNAi") is a phenomenon in which, when cells or the like are introduced with double-stranded RNA (hereinafter abbreviated as "dsRNA") that comprises a sense RNA homologous to the mRNA of a target gene and an antisense RNA complementary to the sense RNA, the dsRNA can induce degradation of the target gene mRNA and suppress the expression of the target gene. As RNAi can be used to suppress target gene expression as described above, it has drawn a great deal of attention as a method applicable to gene therapy or as a simple gene knockout method replacing conventional methods of gene disruption, which are based on complicated and inefficient homologous recombination. The RNAi phenomenon was originally found in Nematode (Fire, A. et al, Nature, 391:806, 1998). Currently, the phenomenon is observed not only in Nematode but also in various organisms, including plants, Nemathelminthes, Drosophila, fruitflies, and protozoa (Fire, A., Trends Genet., 15:358, 1999; Sharp, P.A., Genes Dev., 15:485, 2001; Hammond, S.M. et al., Nature Rev. Genet., 2:110, 2001; Zamore, P.D., Nat. Struct. Biol., 8:746, 2001). It has been confirmed that target gene expression is actually suppressed when introducing exogenous dsRNA into these organisms. RNAi is also being used as a method for creating knockout individuals.

In mammalian cells, like other organisms, there have been attempts to induce RNAi by introducing exogenous dsRNA. In this case, however, the defense mechanism of the host cell against viral infection operated by the introduced dsRNA, and thus protein synthesis was inhibited, and no RNAi was observed. However, it was reported that, when short dsRNA having a full length of 21 or 22 base pairs (bp), which comprises a single stranded 3' overhang of 2 or 3 nucleotides (nt), was introduced into mammalian cells in place of long double-stranded RNAs which are used in other organisms, RNAi could be induced in the mammalian cells (Elbashir, S.M. et al., Nature, 411:494, 2001; Caplen, N.J. et al., Proc. Natl. Acad. Sci. USA, 98:9742, 2001).

In addition, it was reported that siRNA molecules having a 2 nt overhang at the 3' end of each of the antisense and sense strands and comprising a 19-bp duplex region were the initiator of RNAi pathway and that either siRNA molecules having blunt ends or siRNA molecules having a duplex region shorter than 19 bp (base pair) showed low efficiency, even when they were tested at high concentrations (Elbashir et al., EMBO J., 20:6877, 2001). Thus, siRNA molecules longer than 19 bp have been tested, whereas the gene silencing efficiency of siRNA molecules shorter than 19 bp has not been tested due to the reports of negative results.

WO 2009/029688 which is prior art according to Article 54(3) EPC discloses asymmetrical duplex RNA molecules that are capable of effecting sequence-specific gene silencing.

Bramsen, Jesper B., et al: "Improved silencing properties using small internally segmented interfering RNAs", Nucleic Acids Research, Oxford University Press, Surrey, GB, vol. 35, no. 17, 1 January 2007, pages 5886-5897 describes a siRNA designed composed of an intact antisense strand complemented with two shorter 10-12nt sense strands. This three-stranded construct, termed small internally segmented interfering RNA (sisiRNA), is highly functional demonstrating that an intact sense strand is not a prerequisite for RNA interference.

Rose S. D., et al: "Functional polarity is introduced by Dicer processing of short substrate RNAs", Nucleic Acids Research, Oxford University Press, Surrey, GB, vol. 33, no. 13, 26 July 2005, pages 4140-4156 discloses the evaluation of the products of in vitro dicing reactions using electrospray ionization mass spectrometry. It was shown that the use of asymmetric duplexes having a single 2-base 3'-overhang restricts the heterogeneity that results from dicing and that the inclusion of DNA residues at the ends of blunt duplexes also limits heterogeneity.

WO 2005/062937 discloses a method of enhancing the ability of a first strand of an RNAi agent to act as a guide strand in mediating RNAi, the RNAi agent derived from an RNA duplex having at least one blunt end comprising lessening the base pair strength between the 5' end of the first strand and the 3' end of a second strand of the duplex as compared to the base pair strength between the 3' end of the first strand and the 5' end of the second strand.

WO 2005/079533 provides methods of conducting RNAi using siRNAs that are sequentially administered as single-stranded oligonucleotides whereby the siRNAs can be canonical or have non-canonical ends.

US 2006/0142228 relates to an isolated siRNA of from about 5 to about 20 nucleotides that mediates RNA interference.

Meanwhile, unexpected problems were found in gene silencing mediated by RNAi molecules. Specifically, the problems are that exogenous siRNA molecules saturate the RNAi machinery. Such saturation leads to the competition between siRNA molecules. For this reason, the efficiency of intracellular miRNA was reduced, and when two or more kinds of siRNA molecules were introduced, the gene silencing efficiency of the siRNAs was reduced. Moreover, in conventional siRNA molecules, the sense strand rather than the antisense strand can act, and thus the risk of off-target effects exists.

Accordingly, the present inventors have made extensive efforts to provide a novel siRNA molecule, which has high gene silencing efficiency and, at the same time, does not interfere with other exogenous or endogenous RNAi machineries. As a result, the present inventors have constructed siRNA molecules having a novel structure, and found that the constructed siRNA molecule has gene silencing efficiency higher than or similar to that of previously known siRNA molecules, does not interfere with other exogenous or endogenous RNAi machineries and does not show off-target effects caused by the sense strand, thereby completing the present invention.

### SUMMARY OF INVENTION

It is an object of the present invention to provide a novel siRNA molecule, which does not saturate the RNAi machinery, thus making it possible to solve the problems associated with the competition between siRNAs, solves the problems associated with the off-target effects resulting from the sense strand of siRNA molecules and, at the same time, has an excellent gene silencing effect.

Another object of the present invention is to provide an in vitro method for inhibiting the expression of a target gene in a cell using said siRNA molecule.

To achieve the above objects, the present invention provides a small interfering RNA molecule (siRNA molecule) comprising: a 19 nucleotide (nt) antisense strand; and a 15-17 nt sense strand having a sequence complementary to the antisense strand, wherein the 5' end of the antisense strand has a blunt end and the 3' end of the antisense strand has an overhang of 2-4 nt.

The present invention also provides an in vitro method for inhibiting the expression of a target gene in a cell using said siRNA molecule.

Other features and aspects of the present invention will be more apparent from the following detailed description and the appended claims.

### BRIEF DESCRIPTION OF DRAWINGS

FIG. 1 is a graphic diagram showing TIG3 mRNA levels according to the structures of TIG3 mRNA-targeting siRNA molecules upon introduction of the siRNAs into cells.
FIG. 2 is a graphic diagram showing mRNA levels according to the structures of lamin mRNA-targeting and survivin mRNA-targeting siRNA molecules upon introduction of the siRNAs into cells.
FIG. 3 is a graphic diagram showing the gene silencing efficiencies of 16+3A siRNA structures targeting the mRNAs of TIG3, Lamin and Survivin.
FIG. 4 is a graphic diagram showing the gene silencing efficiency of siRNA molecules consisting of a 21 nucleotides (nt) antisense strand which target the mRNAs of TIG3, lamin and survivin.
FIG. 5 is a graphic diagram showing mRNA levels upon introduction of 15+4S and 17+2S siRNA structures into cells in comparison with 15+4A and 17+2A siRNA structures.
FIG. 6 is a graphic diagram showing mRNA levels upon introduction of 17-2A and 15-4A siRNA structures into cells in comparison with 17+2A and 15+4A siRNA structures.
FIG. 7 is a graphic diagram showing the gene silencing efficiencies of 17+2A and 16+3A siRNA structures targeting Integrin mRNA.
FIG. 8 is a graphic diagram showing the IC₅₀ values for TIG3, Lamin, Survivin and Integrin siRNAs.
FIG. 9 shows 16+5A siRNA structures for various genes.
FIG. 10 is a graphic diagram showing the mRNA level of each gene of FIG. 9 upon introduction of siRNA into cells.
FIG. 11 is a photograph showing the results of Western blotting conducted to examine whether the expression of Survivin gene and the *NF-kB* gene is silenced upon introduction of a 16+3A or 16+5A siRNA structure into cells.
FIG. 12 is a fluorescence photograph and a graphic diagram, which show the phenotype of cells treated with siRNAs.
FIG. 13 shows the results obtained by introducing 19+2 and 16+3A siRNA structures into cells and subjecting the cells to 5' RACE analysis.
FIG. 14 shows a photograph and a graphic diagram, which show the results obtained by introducing 19+2 and 16+3A siRNA structures into cells and then measuring the sensitivity of the siRNAs to serum nucleases.
FIG. 15 is a graphic diagram showing CREB3 mRNA levels according to the structures of siRNAs upon introduction of each of siTIG, siSurvivin and siLamin into cells together with siCREB3.
FIG. 16 is a graphic diagram showing the ratio of the standardized luciferase activity of a miR-21 target site-containing reporter to the standardized luciferase activity of a reporter containing no miR-21 target site according to the structures of siRNAs upon introduction of siTIG3.
FIG. 17 illustrates sense target mRNA and antisense target mRNA when an experiment of off-target effects is designed.
FIG. 18 is a graphic diagram showing the inhibitory effects of luciferase expression by the sense strand and antisense strand of siRNAs according to the structures of siRNAs in an experiment of off-target effects.
FIG. 19 is a graphic diagram showing a comparison of the off-target effects of the sense strands of 16+3 and 16+3A siRNA structures.
FIG. 20 is a graphic diagram showing the off-target effects of the sense strand of 19+2 and 16+3A siRNA structures, the 5' end of the sense strand of which has been modified, and showing CREB3 mRNA levels according to the structures of siRNAs upon introduction of the siTIG structure into cells together with siCREB3.

### DETAILED DESCRIPTION OF THE INVENTION,

### AND PREFERRED EMBODIMENTS

All scientific and technical terms used in the detailed description of the invention and the like are defined as follows. As used herein, "siRNA" is a short double-stranded RNA (dsRNA) that mediates efficient gene silencing in a sequence-specific manner.

The term "endogenous gene" refers to a native gene in its original location in the genome of a cell. In contrast, the term "transgene" refers to either a gene derived from an exogenous source, such as a virus or an intracellular parasite, or a gene introduced by recombinant techniques or other physical methods.

The term "gene" must be regarded in the broadest sense, and a target gene may encode a structural protein or a regulatory protein. The term "regulatory protein" includes a transcription factor, a heat shock protein or a protein involved in DNA/RNA replication, transcription and/or translation. The target gene may also be resident in a viral genome which has integrated into the animal gene or is present as an extrachromosomal element. For example, the target gene may be a gene on an HIV genome. In this case, the siRNA molecule is useful in inactivating translation of the HIV gene in a mammalian cell.

The term "nucleotide (nt)" refers to the basic unit of nucleic acid, and the term "19 nt nucleic acid" refers to a single-stranded nucleic acid of 19 nucleotides.

The present invention relates to a double stranded small interfering RNA molecule (siRNA molecule) comprising: a 19 nucleotide (nt) antisense strand; and a 15-17nt sense strand having a sequence complementary to the antisense strand, wherein the 5' end of the antisense strand has a blunt end and the 3' end of the antisense strand has an overhang of 2-4 nt.

In the present invention, the siRNA molecule may be chemically or enzymatically synthesized.

In other words, the siRNA structures according to the present invention include so-called "17+2A", "16+3A" and "15+4A" structures, and each of the structures is as follows. The term "17+2A siRNA structure" refers to a double-stranded siRNA molecule comprising a 19 nt antisense strand and a 17 nt sense strand having a sequence complementary thereto, wherein the 5' end of the antisense strand is a blunt end and the 3'-end of the antisense strand has a 2 nt overhang. Also, the term "16+3A siRNA structure" refers to a double-stranded siRNA molecule comprising a 19 nt antisense strand and a 16 nt sense strand having a sequence complementary thereto, wherein the 5' end of the antisense strand is a blunt end and the 3' end of the antisense strand has a 3 nt overhang. Finally, the term "15+4A siRNA structure" refers to a double-stranded siRNA molecule comprising a 19 nt antisense strand and a 15 nt strand having a sequence complementary thereto, wherein the 5'end of the antisense strand is a blunt end and the 3' end of the antisense strand has a 4 nt overhang.

The siRNA structures according to the present invention have the effect of efficiently inhibiting the expression of a target gene without saturating the RNAi machinery. Also, they have the effect of eliminating off-target effects resulting from the sense strand of siRNA.

The siRNA molecule according to the present invention may have the 15+4A structure in which the length of the sense strand is 15 nt and the overhang length of the 3' end of the antisense strand is 4 nt. The 15+4A siRNA structure does not significantly compete with other siRNAs, shows high gene silencing efficiency and minimizes side effects upon gene silencing of siRNAs. Namely, this structure does not saturate the RNAi machinery and minimizes off-target effects resulting from the sense strand of siRNA.

In addition, the siRNA molecule according to the present invention may have the 16+3A structure in which the length of the sense strand is 16 nt and the length of the 3' overhang of the antisense strand is 3 nt. The 16+3A siRNA structure does not significantly complete with other siRNAs, shows high gene silencing efficiency, and minimizes off-target effects resulting from the sense strand of siRNA.

Meanwhile, the siRNA molecule of the present invention may be a molecule synthesized according to a general method, but the scope of the present invention is not limited thereto. Namely, in the present invention, the siRNA molecule may be chemically or enzymatically synthesized. The siRNA molecule of the present invention may be derived from naturally occurring genes by standard recombinant techniques, the only requirement being that the siRNA molecule is substantially complementary at the nucleotide sequence level to at least a part of mRNA of the target gene, the expression of which is to be modified. By "substantially complementary" is meant that the sequence of the antisense strand of the synthesized siRNA is at least about 80%-90% complementary to the mRNA of the target gene, more preferably at least about 90-95% complementary to the mRNA of the target gene, and even more preferably at least about 95-99% complementary or completely complementary to the mRNA of the target gene.

In another aspect, the present invention relates to an in vitro method for inhibiting the expression of a target gene in a cell using said siRNA molecule.

In the present invention, the antisense strand of the siRNA molecule is preferably complementary to the mRNA sequence of a target gene. In the present invention, the target gene may be an endogenous gene or a transgene gene.

### Examples

Hereinafter, the present invention will be described in further detail with reference to examples. It is to be understood, however, that these examples are for illustrative purposes only and are not to be construed to limit the scope of the present invention.

Particularly, in the following examples, only *TIG3, LaminA*/*C, Survivin, Integrin, Calcineurin, ATF6, DBP, TEF, HIF-1α-01, HIF-1α-02* and *NF-kB* were illustrated as target genes, but it is to be understood that, when siRNA molecules targeting other genes are prepared, they will show the same results as those of the siRNA molecules targeting the illustrated genes.

### Example 1: Preparation of siRNA molecules: siRNAs targeting mRNA of TIG3

A variety of siRNA structural variants targeting mRNA of *TIG3* gene that is a tumor suppressor gene known to suppress and regulate protein expression in several human tumor cell lines were prepared. The sequences of the prepared siRNA variants are shown in Table 1 below.

**Table 1: siRNA molecules targeting mRNA of TIG3**

| | Structure | | Sequence | SEQ ID NO |
|---|---|---|---|---|
| (a) | 19+2 | antisense | 5'-UAGAGAACGCCUGAGACAG(dTdT) | 1 |
| | | sense | 3'-(dTdT)AUCUCUUGCGGACUCUGUC | 2 |
| (b) | 19+0 | antisense | 5'-UAGAGAACGCCUGAGACAG | 3 |
| | | sense | 3'-AUCUCUUGCGGACUCUGUC | 4 |
| (c) | 17+0 | antisense | 5'-UAGAGAACGCCUGAGAC | 5 |
| | | sense | 3'-AUCUCUUGCGGACUCUG | 6 |
| (d) | 17+2A | antisense | 5'-UAGAGAACGCCUGAGACAG | 3 |
| | | sense | 3'-AUCUCUUGCGGACUCUG | 6 |
| (e) | 16+0 | antisense | 5'-UAGAGAACGCCUGAGA | 7 |
| | | sense | 3'-AUCUCUUGCGGACUCU | 8 |
| (f) | 16+3 A | antisense | 5'-UAGAGAACGCCUGAGACAG | 3 |
| | | sense | 3'-AUCUCUUGCGGACUCU | 8 |
| (g) | 15+0 | antisense | 5'-UAGAGAACGCCUGAG | 9 |
| | | sense | 3'-AUCUCUUGCGGACUC | 10 |
| (h) | 15+4A | antisense | 5'-UAGAGAACGCCUGAGACAG | 3 |
| | | sense | 3'-AUCUCUUGCGGACUC | 10 |
| (i) | 13+0 | antisense | 5'-UAGAGAACGCCUG | 11 |
| | | sense | 3'-AUCUCUUGCGGAC | 12 |
| (j) | 13+6A | antisense | 5'-UAGAGAACGCCUGAGACAG | 3 |
| | | sense | 3'-AUCUCUUGCGGAC | 12 |

As shown in (a) of Table 1, a structure having a 2 nt 3' overhang at 19-bp (base pair) double-strand, which is known in the prior art as the structure of a siRNA molecule having the most excellent gene silencing effect, was named a "19+2" structure in order to simply express it in comparison with "17+2A", "16+3A" and "15+4A", which are siRNA structures according to the present invention. Also, as shown in (b), (c), (e), (g) and (i) of Table 1, blunt-ended double-stranded RNA structures having lengths of 19 bp, 17 bp, 16 bp, 15 bp and 13 bp were named a "19+0 structure", a "17+0 structure", a "16+0 structure", a "15+0 structure" and a "13+0 structure", respectively. In addition, as shown in (j) of Table 1, a double-stranded siRNA molecule comprising a 19 nt antisense strand and a 13 nt sense strand having a sequence complementary thereto, in which the 5' end of the antisense strand is a blunt end and the 3' end of the antisense strand has a 6 nt overhang, was named a "13+6A structure".

### Example 2: Analysis of gene (TIG3) silencing efficiency of siRNA

The siRNAs prepared in Example 1 and having the structures shown in Table 1, were introduced into a human glioblastoma cell line (ACTC CRL1690) using Lipofectaminc 2000 (Invitrogen). The cells were treated with each of the siRNAs at varying concentrations of 100 nM, 10 nM and 1 nM, and the *TIG3* gene silencing efficiency of each siRNA was measured using quantitative real-time reverse transcription-polymerase chain reaction (RT-PCR). The cells were collected 48 hours after introduction of the siRNA, and the total RNA was extracted from the cell lysate using Tri-reagent Kit (Ambion). Then, the total RNA (1 µg) was used as a template for cDNA synthesis, and the cDNA synthesis was performed using the Improm-Il™ Reverse Transcription System (Promega) according to the manufacturer's protocol. The fraction (1/20) of the cDNA product was analyzed by quantitative real-time RT-PCR using Rotor-Gene 3000 (Corbett Research). The data were analyzed using Rotor-Gene 6 software (Corbett Research), and the primer sequences used in the RT-PCR were as follows.

### Primer pair for RT-PCR of TIG3

TIG3-forward (SEQ ID NO: 13): 5'- AGA TTT TCC GCC TTG GCT AT-3'
TIG3-reverse (SEQ ID NO: 14): 5'- TTT CAC CTC TGC ACT GTT GC-3'

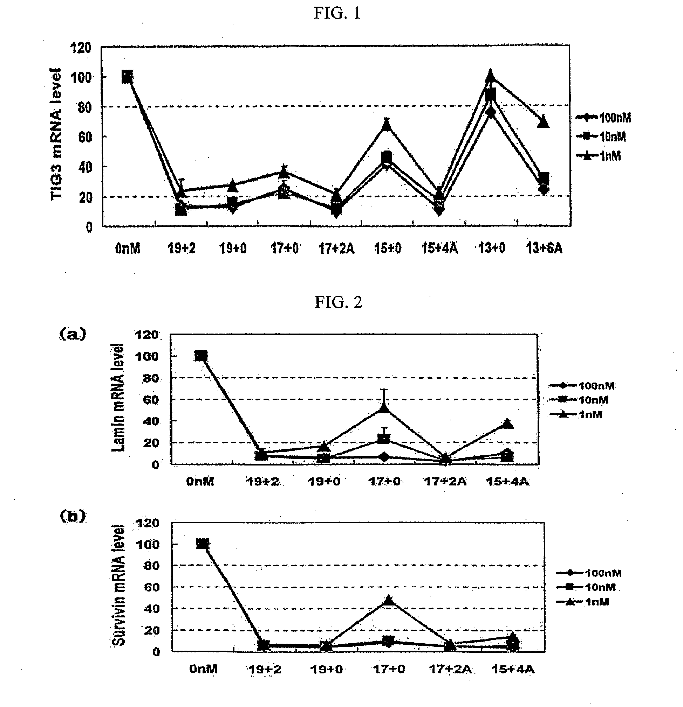

As a result, as shown in FIG. 1, the TIG3 mRNA level was higher in the groups treated with the blunt-ended double-stranded siRNAs having the 19+0, 17+0, 15+0 and 13+0 structures than in the group treated with the 19+2 structure known as the most efficient structure in the prior art, suggesting that the *TIG3* gene silencing effects of the 19+0, 17+0, 15+0 and 13+0 structures were lower than that of the 19+2 structure. Particularly, the 13+0 structure was shown to have little or no gene silencing effect, and the 15+0 structure reduced the *TIG3* mRNA level to 40%, when the cells were treated with the siRNA at concentrations of 100 nM and 10 nM, but it showed a very low gene silencing effect, when the cells were treated with the siRNA at a concentration of 1 nM.

In contrast, in the cases of the 17+2A and 15+4A siRNA structures according to the present invention, *TIG3* mRNA levels almost similar to that of the 19+2 siRNA structure known to have the highest gene silencing efficiency in the prior art were observed, even though the double-stranded region was shorter than 19 nucleotides (nt). However, in the 13+6A structure, a less decrease in the mRNA level was observed.

The above-described experimental results are contrary to prior reports that the gene silencing effect of siRNA structures shorter than 19 nt is lower than that of the previously known 19+2 structure. Namely, the experimental results indicate that, when siRNAs are constructed such that the 3' ends of the antisense strands have 2 nt and 4 nt overhangs, respectively, and the 5' ends of the antisense strands are blunt ends, they show gene silencing efficiency almost equal to that of the 19+2 structure known to have the highest gene silencing efficiency in the prior art, even though the double-stranded regions thereof are as short as 17 nt and 15 nt. However, the 13+6A structure showed low gene silencing efficiency, and thus it could be confirmed that the 17+2A, 16+3A and 15+4A structures are preferred siRNA structures.

In the following examples, additional experiments were carried out using other mRNAs as targets in order to examine whether or not the above-mentioned results are limited only to the *TIG3* gene.

### Example 3: Analysis of gene (LaminA/C and Survivin)-silencing efficiencies of siRNA

As shown in Tables 2 and 3, siRNAs targeting the mRNAs of LaminA/C and Survivin were prepared to have a 19+2 structure, a 19+0 structure, a 17+0 structure, a 17+2A structure and a 15+4A structure. Table 2 shows siRNA molecules targeting LaminA/C mRNA, and Table 3 shows siRNA molecules targeting Survivin mRNA.

**Table 2: siRNA molecules targeting LaminA/C mRNA**

| | Structure | | Sequence | SEQ ID NO |
|---|---|---|---|---|
| (a) | 19+2 | antisense | 5'-UGUUCUUCUGGAAGUCCAG(dTdT) | 15 |
| | | sense | 3'-(dTdT)ACAAGAAGACCUUCAGGUC | 16 |
| (b) | 19+0 | antisense | 5'-UGUUCUUCUGGAAGUCCAG | 17 |
| | | sense | 3'-ACAAGAAGACCUUCAGGUC | 18 |
| (c) | 17+0 | anti sense | 5'-UGUUCUUCUGGAAGUCC | 19 |
| | | sense | 3'-ACAAGAAGACCUUCAGG | 20 |
| (d) | 17+2A | antisense | 5'-UGUUCUUCUGGAAGUCCAG | 17 |
| | | sense | 3'-ACAAGAAGACCUUCAGG | 20 |
| (e) | 15+4A | antisense | 5'-UGUUCUUCUGGAAGUCCAG | 17 |
| | | sense | 3'-ACAAGAAGACCUUCA | 21 |

**Table 3: siRNA molecules targeting Survivin mRNA**

| | Structure | | Sequence | SEQ ID NO |
|---|---|---|---|---|
| (a) | 19+2 | antisense | 5'-UGAAAAUGUUGAUCUCCUU(dTdT) | 22 |
| | | sense | 3'-(dTdT)ACUUUUACAACUAGAGGAA | 23 |
| (b) | 19+0 | antisense | 5'-UGAAAAUGUUGAUCUCCUU | 24 |
| | | sense | 3'-ACUUUUACAACUAGAGGAA | 25 |
| (c) | 17+0 | antisense | 5'-UGAAAAUGUUGAUCUCC | 26 |
| | | sense | 3'-ACUUUUACAACUAGAGG | 27 |
| (d) | 17+2A | antisense | 5'-UGAAAAUGUUGAUCUCCUU | 24 |
| | | sense | 3'-ACUUUUACAACUAGAGG | 27 |
| (e) | 15+4A | antisense | 5'-UGAAAAUGUUGAUCUCCUU | 24 |
| | | sense | 3'-ACUUUUACAACUAGA | 28 |

Each of the prepared siRNAs was introduced into HeLa cells (ACTC CCL-2) at varying concentrations of 100 nM, 10 nM and 1 nM using Lipofectamine 2000 (Invitrogen), and the mRNA levels in the cells were measured using quantitative real-time reverse transcription-polymerase chain reaction (RT-PCR) in the same manner as in Example 2.

### Primer pair for RT-PCR of LaminA/C

Lamin-forward (SEQ ID NO: 29): 5'-CCG AGT CTG AAG AGG TGG TC-3'
Lamin-reverse (SEQ ID NO: 30): 5'-AGG TCA CCC TCC TTC TTG GT-3

### Primer pair for RT-PCR of Survivin

Survivin-forward (SEQ ID NO: 31): 5'-GCA CCA CTT CCA GGG TTT AT-3'
Survivin-reverse (SEQ ID NO: 32): 5'-CTC TGG TGC CAC TTT CAA GA-3'

As a result, as shown in FIG. 2, the siRNA having the 17+2A structure very efficiently reduced the levels of LaminA/C and Survivin mRNA at all the tested concentrations, and it showed efficiency higher than that of the 19+2 structure. The 15+4A structure showed the same gene silencing efficiency as that of the 19+2 structure at concentrations of 100 nM and 10 nM, and the gene silencing efficiency thereof was slightly lower than that of the 19+2 or 17+2A structure at 1 nM.

These results suggest that, when siRNAs targeting other genes are prepared, the 17+2A structure and 15+4A structure according to the present invention have gene silencing efficiency almost equal to that of the 19+2 structure as described in Example 2. In addition, these results indicate that the siRNA structures according to the present invention are not limited only to certain genes and may generally be applied to a wide range of genes.

### Example 4: Analysis of gene silencing efficiency of siRNA: experiment of 16+3A structure

The 16+3A siRNA molecules targeting the mRNAs of TIG3, LaminA/C and Survivin were tested at varying concentrations of 1 nM and 10 nM in the same manner as in Examples 2 and 3, and the mRNA levels of the genes were measured. 16+3A siRNA structures targeting the mRNAs of LaminA/C and Survivin were prepared, and the sequences thereof are as follows.

### Structure of LaminA/C 16+3A

Antisense: 5' UGUUCUUCUGGAAGUCCAG (SEQ ID NO 17)
Sense: 3' ACAAGAAGACCUUCAG (SEQ ID NO 33)

### Structure of Survivin 16+3A

Antisense: 5' UGAAAAUGUUGAUCUCCUU (SEQ ID NO 24)
Sense: 3' ACUUUUACAACUAGAG (SEQ ID NO 34)

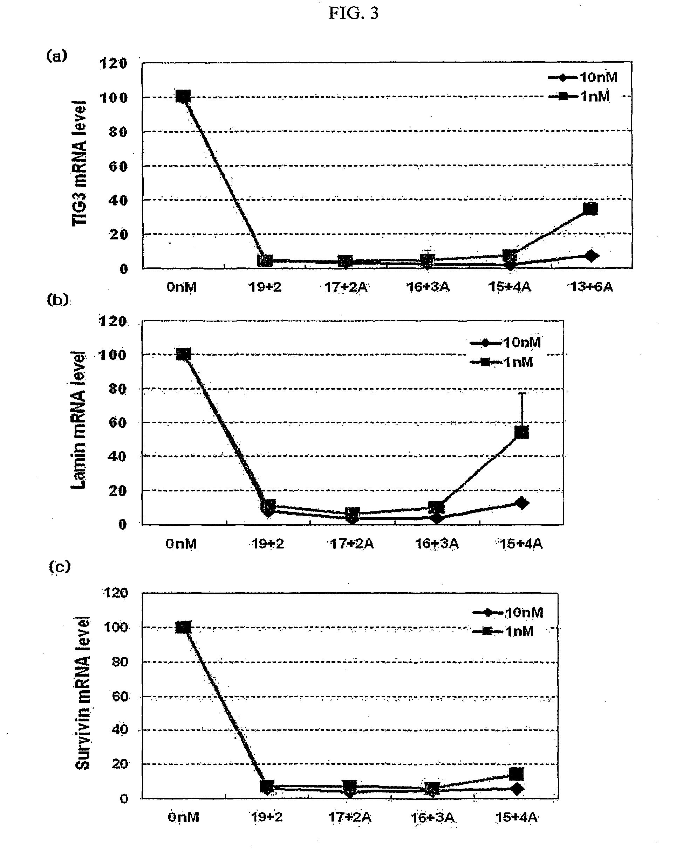

As a result, as shown in (a), (b) and (c) of FIG. 3, when the mRNA levels of TIG3, LaminA/C and Survivin were measured, the introduction of the 16+3A siRNA structure showed the mRNA levels similar to those of the 19+2 structure or 17+2A structure. These experimental results suggest that the 16+3A siRNA structure has gene silencing efficiency similar to that of the 17+2A structure than that of the 15+4A structure.

### Example 5: Analysis of gene silencing efficiency of siRNA consisting of 21 nt antisense strand

siRNA structures targeting the mRNAs of TIG3, LaminA/C and Survivin were prepared such that the length of the antisense strand was 21 nt. The prepared siRNA structures are shown in Tables 4 to 6.

**Table 4: siRNA molecules targeting TIG3 mRNA**

| | Structure | | Sequence | SEQ ID NO |
|---|---|---|---|---|
| (a) | 19+2 | antisense | 5'-UAGAGAACGCCUGAGACAG(dTdT) | 1 |
| | | sense | 3'-(dTdT)AUCUCUUGCGGACUCUGUC | 2 |
| (b) | 19+2A | antisense | 5'-UAGAGAACGCCUGAGACAG(dTdT) | 1 |
| | | sense | 3'-AUCUCUUGCGGACUCUGUC | 4 |
| (c) | 17+4A | antisense | 5'-UAGAGAACGCCUGAGACAG(dTdT) | 1 |
| | | sense | 3'-AUCUCUUGCGGACUCUG | 6 |
| (d) | 16+5A | antisense | 5'-UAGAGAACGCCUGAGACAG(dTdT) | 1 |
| | | sense | 3'-AUCUCUUGCGGACUCU | 8 |
| (e) | 15+6A | antisense | 5'-UAGAGAACGCCUGAGACAG(dTdT) | 1 |
| | | sense | 3'-AUCUCUUGCGGACUC | 10 |
| (f) | 14+7A | antisense | 5'-UAGAGAACGCCUGAGACAG(dTdT) | 1 |
| | | sense | 3'-AUCUCUUGCGGACU | 35 |
| (g) | 13+8A | antisense | 5'-UAGAGAACGCCUGAGACAG(dTdT) | 1 |
| | | sense | 3'-AUCUCUUGCGGAC | 12 |

**Table 5: siRNA molecules targeting LaminA/C mRNA**

| | Structure | | Sequence | SEQ ID NO |
|---|---|---|---|---|
| (a) | 19+2 | antisense | 5'-UGUUCUUCUGGAAGUCCAG(dTdT) | 15 |
| | | sense | 3'-(dTdT)ACAAGAAGACCUUCAGGUC | 16 |
| (b) | 19+2A | antisense | 5'-UGUUCUUCUGGAAGUCCAG(dTdT) | 15 |
| | | sense | 3'-ACAAGAAGACCUUCAGGUC | 18 |
| (c) | 17+4A | antisense | 5'-UGUUCUUCUGGAAGUCCAG(dTdT) | 15 |
| | | sense | 3'-ACAAGAAGACCUUCAGG | 20 |
| (d) | 16+5A | antisense | 5'-UGUUCUUCUGGAAGUCCAG(dTdT) | 15 |
| | | sense | 3'-ACAAGAAGACCUUCAG | 33 |
| (e) | 15+6A | antisense | 5'-UGUUCUUCUGGAAGUCCAG(dTdT) | 15 |
| | | sense | 3'-ACAAGAAGACCUUCA | 21 |

**Table 6: siRNA molecules targeting Survivin mRNA**

| | Structure | | Sequence | SEQ ID NO |
|---|---|---|---|---|
| (a) | 19+2 | antisense | 5'-UGAAAAUGUUGAUCUCCUU(dTdT) | 22 |
| | | sense | 3'-(dTdT)ACUUUUACAACUAGAGGAA | 23 |
| (b) | 19+2A | antisense | 5'-UGAAAAUGUUGAUCUCCUU(dTdT) | 22 |
| | | sense | 3'-ACUUUUACAACUAGAGGAA | 25 |
| (c) | 17+4A | antisense | 5'-UGAAAAUGUUGAUCUCCUU(dTdT) | 22 |
| | | sense | 3'-ACUUUUACAACUAGAGG | 27 |
| (d) | 16+5A | antisense | 5'-UGAAAAUGUUGAUCUCCUU(dTdT) | 22 |
| | | sense | 3'-ACUUUUACAACUAGAG | 34 |
| (e) | 15+6A | antisense | 5'-UGAAAAUGUUGAUCUCCUU(dTdT) | 22 |
| | | sense | 3'-ACUUUUACAACUAGA | 28 |

Each of the prepared siRNAs was introduced into HeLa cells (ACTC CCL-2) at varying concentrations of 10 nM and 1 nM using Lipofectamine 2000 (Invitrogen), and the mRNA levels of the genes were measured using quantitative real-time reverse transcription-polymerase chain reaction (RT-PCR) in the same manner as in Examples 2 and 3.

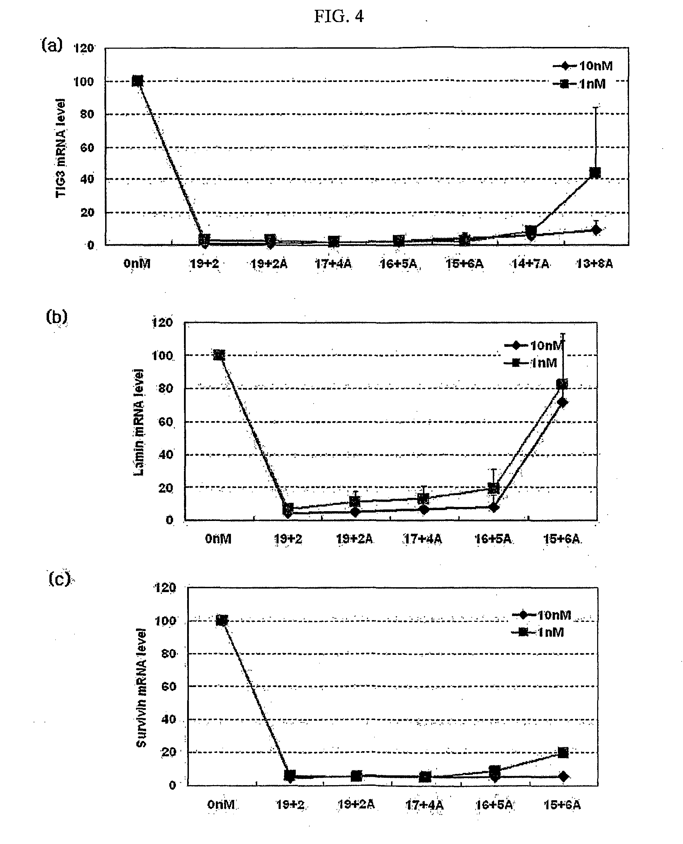

First, each of the siRNAs was introduced into the cells, and the mRNA levels were measured. As a result, as shown in (a) of FIG. 4, when each of the 19+2A, 17+4A, 16+5A and 15+6A siRNA structures was introduced into the cells at a concentration of 10 nM, the TIG3 mRNA-silencing efficiency of each siRNA structure was almost equal to that of the prior 19+2 structure. Also, TIG3 mRNA silencing efficiency was gradually decreased in the 14+7A structure and the 13+8A structure. Moreover, when each siRNA structure was introduced at a concentration of 1 nM, the TIG3 mRNA silencing efficiency of the siRNA structures was greatly decreased in the 14+7A and 13+8A structures.

The experimental results indicate that, when the length of the antisense strand of the siRNA molecule was increased to 21 nt, the siRNA molecule shows high gene silencing efficiency, if it is constructed such that the 5' end of the antisense strand is a blunt end and the 3' end of the antisense strand has an overhang. However, the 14+7A structure or the 13+8A structure show reduced gene silencing efficiency, suggesting that the preferred length of the sense strand of the siRNA molecule is 15-19 nt.

In addition, the mRNA levels of LaminA/C and Survivin were measured. As a result, as shown in (b) and (c) of FIG. 4, when the siRNAs of Table 5 were introduced into cells, the gene silencing efficiency of the 15+6A structure was decreased, but the 19+2A structure, the 17+4A structure and the 16+5A structure showed high gene silencing efficiency. Also, when the siRNAs of Table 6 were introduced into the cells, treatment of the cells with 1 nM of the 15+6A structure showed a slight decrease in gene silencing efficiency, but treatment of the cells with 10 nM of each of the 15+6A structure, the 19+2A structure, the 17+4A structure and the 16+5A structure showed gene silencing efficiency almost equal to that of the prior 19+2 structure.

These experimental results indicate that, when the 19+2A, 18+3A, 17+4A and 16+5A structures targeting genes other than the *TIG3* gene are prepared, the siRNA structures have gene silencing efficiency almost equal to that of the prior 19+2 structure. This suggests that the siRNA structures according to the present invention are not limited to certain genes and may generally be applied to a wide range of genes.

### Example 6: Orientation I of blunt end and overhang

In order to examine the effect of the orientation of blunt end and overhang of the inventive siRNA structures on gene silencing efficiency, siRNA molecules having the structures shown in Tables 7 and 8 were prepared and tested in the same manner as in Example 2 (TIG3) and Example 3 (LaminA/C).

**Table 7: siRNA molecules targeting TIG3 mRNA**

| | Structure | Structure | Sequence | SEQ ID NO |
|---|---|---|---|---|
| (a) | 19+2 | antisense | 5'-UAGAGAACGCCUGAGACAG(dTdT) | 1 |
| | | sense | 3'-(dTdT)AUCUCUUGCGGACUCUGUC | 2 |
| (b) | 15+4A | antisense | 5'-UAGAGAACGCCUGAGACAG | 3 |
| | | sense | 3'-AUCUCUUGCGGACUC | 10 |
| (c) | 15+4S | antisense | 5'-GAACGCCUGAGACAG | 36 |
| | | sense | 3'-AUCUCUUGCGGACUCUGUC | 4 |

**Table 8: siRNA molecules targeting LaminA/C) mRNA**

| | Structure | | Sequence | SEQ ID NO |
|---|---|---|---|---|
| (a) | 19+2 | antisense | 5'-UGUUCUUCUGGAAGUCCAG(dTdT) | 15 |
| | | sense | 3'-(dTdT)ACAAGAAGACCUUCAGGUC | 16 |
| (b) | 17+2A | antisense | 5'-UGUUCUUCUGGAAGUCCAG | 15 |
| | | sense | 3'-ACAAGAAGACCUUCAGG | 20 |
| (c) | 17+2S | antisense | 5'-UUCUUCUGGAAGUCCAG | 37 |
| | | sense | 3'-ACAAGAAGACCUUCAGGUC | 18 |

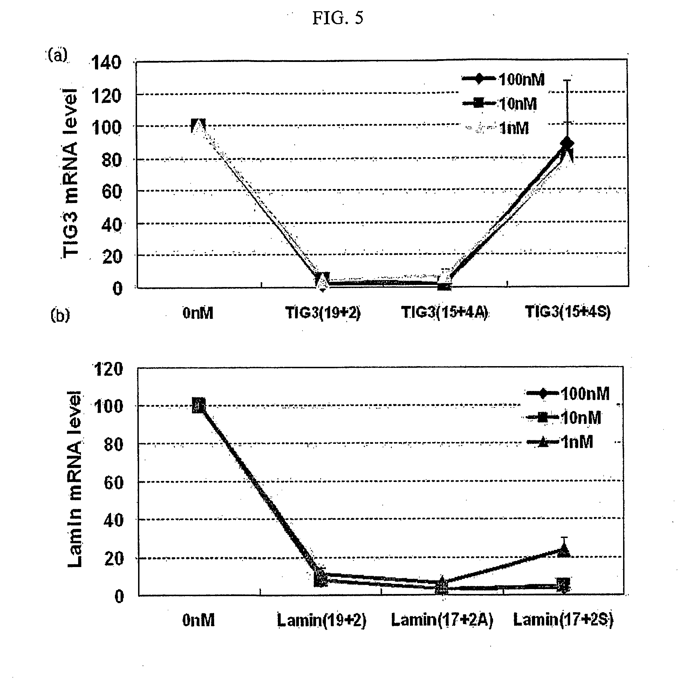

As a result, as shown in (a) of FIG. 5, when the siRNA molecules of Table 7 that target TIG mRNA were introduced into cells, the 15+4S structure shown in (c) of Table 7 showed an mRNA level much higher than that of the 15+4A structure shown in (b) of Table 7. Also, the siRNA molecules shown in Table 8 were introduced in cells and then observed for Lamin mRNA levels. As a result, as shown in (b) of FIG. 5, when each of the siRNAs was introduced at a concentration of 1 nM, the 17+2S structure showed an mRNA level slightly higher than that of the 17+2A structure.

These experimental results indicate that, when siRNA structures are constructed such that the 3' end of the antisense strand is a blunt end, the length of the antisense strand is 15-17 nt and the length of the sense strand is 19 nt, the gene silencing efficiency of the siRNA structures is decreased. In order words, it could be seen that the siRNA structures in which the 5' end of the antisense strand is a blunt end and the 3' end of the antisense strand has an overhang showed higher gene silencing efficiency. However, because this may be an effect resulting from the change in the length of the antisense strand, the following experiment was additionally carried out.

### Example 7: Orientation II of blunt end and overhang

siRNA molecules having the structures shown in Tables 9 and 10 were prepared and tested in the same manner as in Example 3 (LaminA/C) and Example 2 (TIG3), and the mRNA levels of the genes were measured.

**Table 9: siRNA molecules targeting LaminA/C mRNA**

| | Structure | | Sequence | SEQ ID NO |
|---|---|---|---|---|
| (a) | 19+2 | antisense sense | 5'-UGUUCUUCUGGAAGUCCAG(dTdT) | 15 |
| | | | 3'- (dTdT)ACAAGAAGACCUUCAGGUC | 16 |
| (b) | 17+0 | antisense sense | 5'-UGUUCUUCUGGAAGUCC | 19 |
| | | | 3'-ACAAGAAGACCUUCAGG | 20 |
| (c) | 17+2A | antisense sense | 5'-UGUUCUUCUGGAAGUCCAG | 15 |
| | | | 3'-ACAAGAAGACCUUCAGG | 20 |
| (d) | 17-2A | antisense sense | 5'-UGUUCUUCUGGAAGUCCAG | 15 |
| | | | 3'-AAGAAGACCUUCAGGUC | 38 |

**Table 10: siRNA molecules targeting TIG3 mRNA**

| | Structure | | Sequence | SEQ ID NO |
|---|---|---|---|---|
| (a) | 19+2 | antisense sense | 5'- UAGAGAACGCCUGAGACAG(dTdT) | 1 |
| | | | 3'- (dTdT)AUCUCUUGCGGACUCUGUC | 2 |
| (b) | 15+0 | antisense sense | 5'-UAGAGAACGCCUGAG | 9 |
| | | | 3'-AUCUCUUGCGGACUC | 10 |
| (c) | 15+4A | antisense sense | 5'- UAGAGAACGCCUGAGACAG | 3 |
| | | | 3'-AUCUCUUGCGGACUC | 10 |
| (d) | 15-4A | antisense sense | 5'-UAGAGAACGCCUGAGACAG | 3 |
| | | | 3'-CUUGCGGACUCUGUC | 39 |

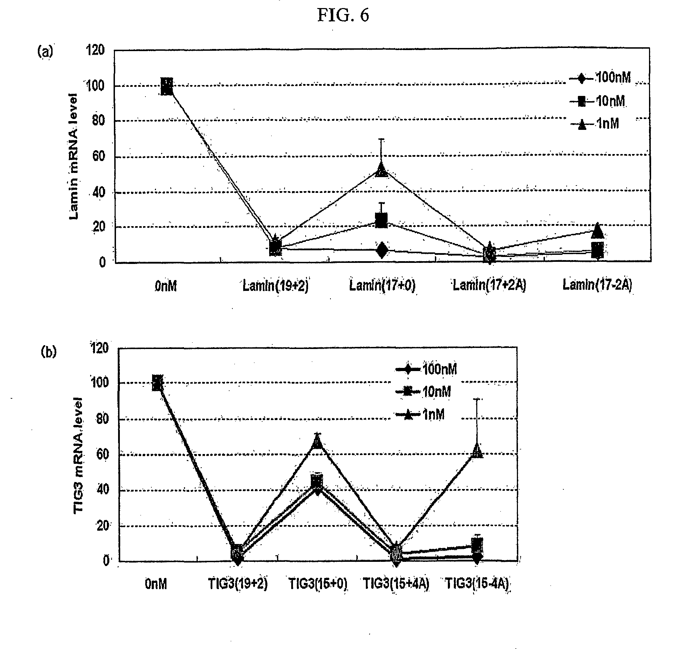

As a result, as shown in FIG. 6, when the 17+2A and 17-2A structures were compared with each other, the 17-2A structure showed higher LaminA/C mRNA levels at all concentrations ((a) of FIG. 6). When the 15+4A and 15-4A structures were compared with each other, the 15-4A structure showed higher TIG3 mRNA levels ((b) of FIG. 6). Particularly, the 15-4A structure showed a very high mRNA level at a concentration of 1 nM.

These experimental results indicate that, when the 5' end of the antisense strand has a blunt end and the 3' end of the antisense strand has an overhang, the siRNA structures show gene silencing efficiency almost similar to that of the 19+2 structure known as the most efficient structure in the prior art, but when the 3' end of the antisense strand is a blunt end and the 5' end of the antisense strand has an overhang, the siRNA has low gene silencing efficiency.

These results together with the results of Example 6 indicate that the orientation of the blunt end and overhang of siRNA structures influences the gene silencing efficiency of the siRNA structures.

### Example 8: Analysis of gene silencing efficiency of Integrin siRNA and comparison of IC₅₀

For the integrin gene, siRNAs having the structures shown in Table 11 were prepared and tested in the same manner as in Example 2 to measure the mRNA levels, and IC₅₀ values obtained from experimental results for the integrin gene and the above-mentioned *TIG3, LaminA*/*C, Survivin* genes were compared with each other.

**Table 11: siRNA molecules targeting Integrin mRNA**

| | Structure | | Sequence | SEQ ID NO |
|---|---|---|---|---|
| (a) | 19+2 | antisense sense | 5'-AUAUCUGAAGUGCAGUUCA(dTdT) | 40 |
| | | | 3'-(dTdT)UAUAGACUUCACGUCAAGU | 41 |
| (b) | 17+2A | antisense sense | 5'-AUAUCUGAAGUGCAGUUCA | 42 |
| | | | 3'-UAUAGACUUCACGUCAA | 43 |
| (c) | 16+3A | antisense sense | 5'-AUAUCUGAAGUGCAGUUCA | 42 |
| | | | 3'-UAUAGACUUCACGUCA | 44 |

### Primer pair for RT-PCR of Integrin

Integrin-forward (SEQ ID NO: 45): 5'-CGT ATC TGC GGG ATG AAT CT-3'
Integrin-reverse (SEQ ID NO: 46): 5' GGG TTG CAA GCC TGT TGT AT-3'

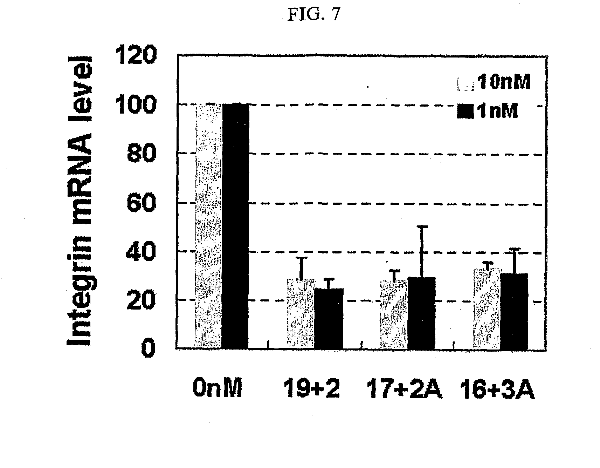

First, the integrin mRNA levels were measured. As a result, as shown in FIG. 7, the 17+2A and 16+3A structures all showed mRNA levels similar to that of the siRNA of 19+2 structure.

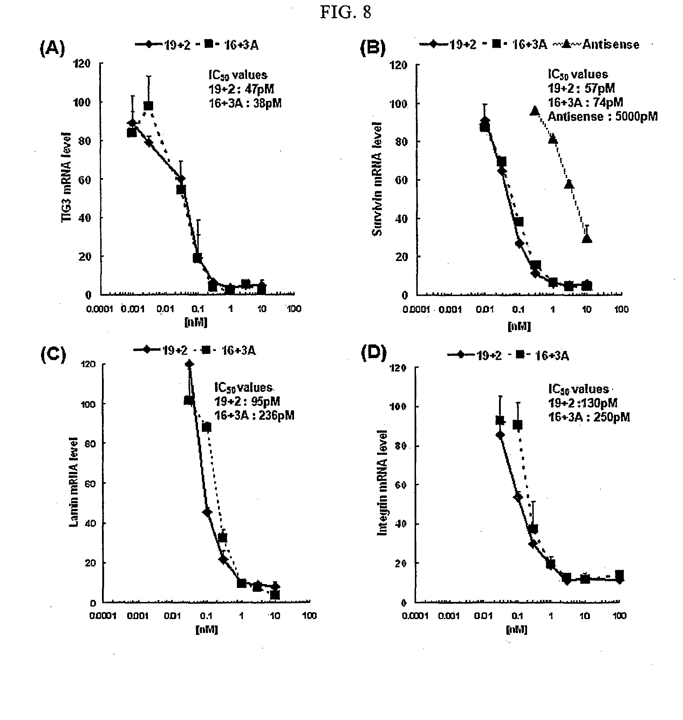

Meanwhile, the IC₅₀ values were measured. As a result, as shown in FIG. 8, the 16+3A siRNA structure showed IC₅₀ values, which were almost equal to those of the 19+2 siRNA structure (siTIG3 and siSurvivin) or slightly increased (siLamin and siIntegrin).

Additionally, in order to examine if RNAi-mediated gene silencing can be performed only by the antisense strand of siRNA, only the siRNA antisense strand was introduced into cells and measured for gene silencing efficiency. Specifically, the siSurvivin antisense strand was introduced into cells in the same manner as described, and the Survivin mRNA level was measured. As a result, as shown in FIG. 8B, when only the antisense strand was introduced, the gene silencing efficiency was significantly reduced.

These experimental results suggest that the gene silencing efficiency of the siRNA molecules according to the present invention arises from dsRNA-mediated RNAi.

### Example 9: Analysis of gene silencing efficiency of 16+5A siRNA structure

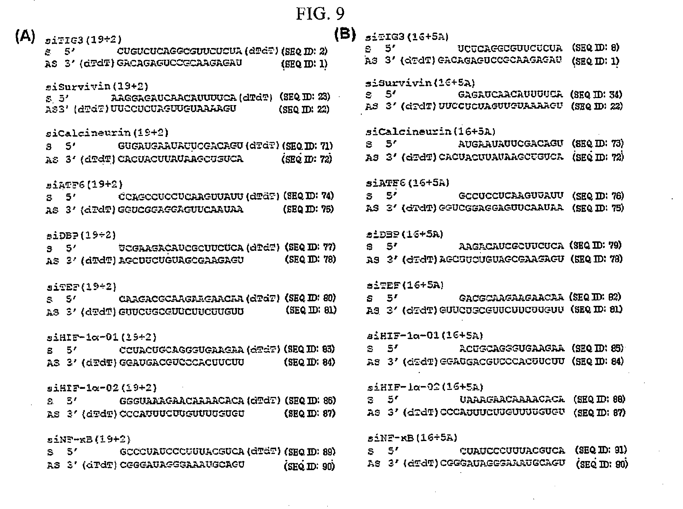

dTdT was added to the 3' end of the antisense strand of the 16+3A structure to construct a 16+5A structure as shown in FIG. 9, and the silencing activities of the 19+2 and 16+3A siRNA structures were compared with each other. At this time, whether the siRNA structures according to the present invention may generally be applied was additionally tested by constructing siRNA structures for various genes in addition to prior *TIG3, LaminA*/*C, Survivin* and *Integrin* genes, measuring the activities of the constructed structures and comparing the measured activities with that of the prior 19+2 structure. The siRNA activities were determined by measuring the mRNA level of each gene through quantitative real-time RT-PCR as described in Example 2. Primer pairs for the *TIG3, LaminA*/*C, Survivin* and *Integrin* genes are presented in Examples as described above, and primer pairs for other genes are as follows.

### Primer pair for RT-PCR of Calcineurin

Calcineurin-forward (SEQ ID NO: 47): 5'-GCA ACC ATG AAT GCA GAC AC-3'
Calcineurin -reverse (SEQ ID NO: 48): 5'-TGG TGA AAG TCC ACC ATG AA-3'

### Primer pair for RT-PCR of ATF6

ATF6-forward (SEQ ID NO: 49): 5'-GCC TTT ATT GCT TCC AGC AG-3'
ATF6-reverse (SEQ ID NO: 50): 5'-TGA GAC AGC AAA ACC GTC TG-3'

### Primer pair for RT-PCR of DBP

DBP-forward (SEQ ID NO: 51): 5'-GTA GAC CTG GAC GCC TTC CT-3'
DBP-reverse (SEQ ID NO: 52): 5'-CGG GTT CAA AGG TCA TCA AC-3'

### Primer pair for RT-PCR of TEF

TEF-forward (SEQ ID NO: 53): 5'-CCC CAG CCT ATG ATC AAA AA-3'
TEF-reverse (SEQ ID NO: 54): 5'-CCG GAT GGT GAT CTG ATT CT-3'

### Primer pair for RT-PCR of HIF1α (HIF1α-01 & HIF1α-02)

HIF1α-forward (SEQ ID NO: 55): 5'-CCA GCA ACA GAA AGT CGT CA-3'
HIF1α-reverse (SEQ ID NO: 56): 5'-GGC TAT ACT TGG GCA TGG AA-3'

### Primer pair for RT-PCR of NF-kB

NF-kB-forward (SEQ ID NO: 57): 5'-CCT GGA GCA GGC TAT CAG TC-3'
NF-kB-reverse (SEQ ID NO: 58): 5'-CAC TGT CAC CTG GAA GCA GA-3'

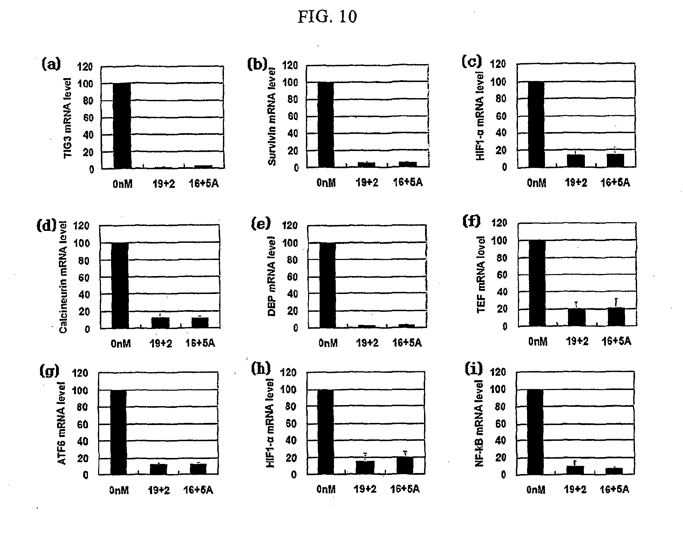

As a result, as shown in (a) to (i) of FIG. 10, when the mRNA level of each gene was measured, the introduction of the 16+5A siRNA structure also showed mRNA levels similar to that of the 19+2 structure. These experimental results suggest that the 16+5A siRNA structure also has high gene silencing efficiency.

### Example 10: Analysis of gene silencing efficiency of siRNA by Western blotting technique

In order to examine the gene silencing efficiency of the siRNA structure according to the present invention, Western blotting was performed for the Survivin gene and the *NF-kB* gene. First, 10 nM of each of 19+2 and 16+3A structures of siSurvivin and 19+2 and 16+5A structures of siNF-kB was introduced into HeLa cells (ACTC CCL-2) using Lipofectamine 2000 (Invitrogen), and after 48 hours, the cells were lysated in RIPA buffer containing 150 mM NaCl, Tris pH 7.5, 0.5% SDS, 0.1% sodium deoxycholate, 0.02% sodium azide, 1 mM EDTA and protease inhibitors.

Then, the obtained protein was electrophoresed on SDS-PAGE gel using Tris-Glycine SDS running buffer, and then transferred to a nitrocellulose membrane. Then, the membrane was blocked with TBS buffer containing 5% milk powder, and then incubated with antibodies (Cell Signaling) to the Survivin protein and the NF-kB protein. The blots were developed with an ECL detection system (Amersham Biosciences) and exposed to an X-ray film (Kodak).

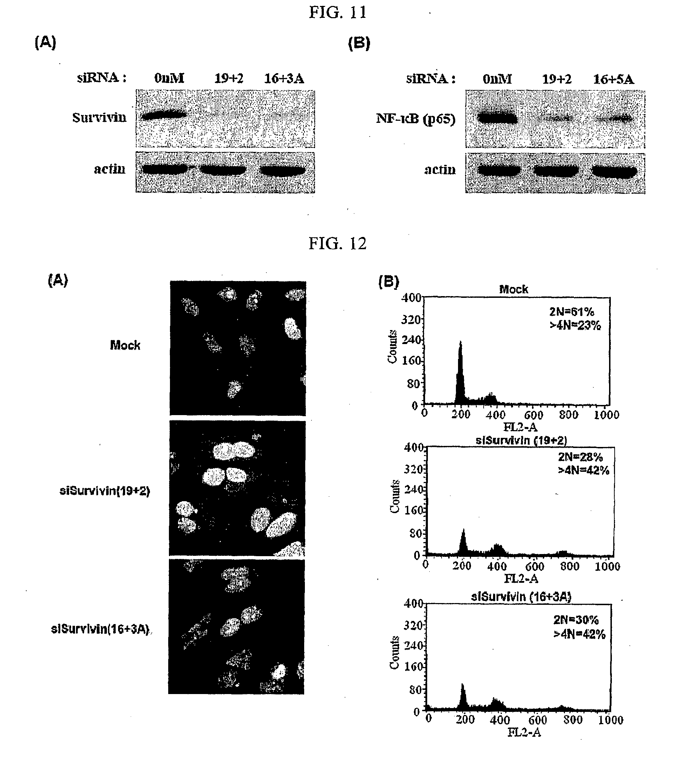

As a result, as shown in FIGS. 11(a) and 11(b), the 16+3A and 16+5A siRNA structures showed gene silencing efficiency similar to that of the prior 19+2 structure and significantly suppressed the expression of each of the proteins compared to the control group (not treated with siRNA).

### Example 11: Analysis of phenotype upon treatment with siRNA

Survivin is an inhibitor of apoptosis protein that is required for cell viability and cell cycle progression. Also, it has been reported that survivin is overexpressed in most cancer cells and, when the function thereof is blocked, cell proliferation is inhibited and a polyploidy phenotype is induced. Accordingly, in this Example, siRNAs for the survivin gene were constructed to have the 19+2 structure and the 16+3A structure according to the present invention, and a change in the phenotype thereof was observed.

First, each of the 19+2 and 16+3A siRNA structures was introduced into HeLa cells using Lipofectamine 2000, and after 48 hours, the cells were fixed with 3.7% formaldehyde. The fixed cells were stained with 2 µg of diamidinophenyl indole (DAPI) solution, and the phenotype thereof was observed with a fluorescence microscope.

As a result, as shown in FIG. 12(A), in comparison with a mock treated with transfection reagent alone without siRNA, the cell group treated with the 19+2 siRNA structure showed increased cell size and an increase in the number of polyploid cells. Also, in the cell group treated with the 16+3 siRNA structure, it was observed that a polyploidy phenotype was induced.

Meanwhile, in order to quantify the ratio of polyploid cells in each of the cell groups, flow cytometry was performed. Specifically, each of the 19+2 and 16+3A siRNA structures was introduced into HeLa cells using Lipofectamine 2000, and after 48 hours, the cells were collected, washed with 2% FBS in PBS (phosphate-buffered saline), and then fixed in 70% ethanol overnight. Then, the cells were suspended in 250 µl of PBS containing 50 µg/ml of RNase A, and incubated at 37°C for 30 minutes, followed by treatment with 25 µl/ml of propidium iodide. The propidium iodide-stained cells were analyzed by FACSCalibur System (Becton Dickinson).

As a result, as shown in 12(B), it was observed that the groups treated with the 19+2 and 16+3A siRNA structures, respectively, all induced polyploid cells at similar ratios.

These results suggest that the 16+3 siRNA structure according to the present invention inhibits mRNA expression at the same level as that of the 19+2 siRNA structure and also induced a change in phenotype in a manner similar to the 19+2 siRNA structure.

### Example 12: Analysis of gene silencing mechanism

In order to examine if the siRNA structure according to the present invention inhibits gene expression by the same mechanism as that of the prior 19+2 siRNA structure, the following experiment was carried out. Specifically, 5'-RACE analysis was carried out in order to analyze cleavage sites in the mRNA of each of the 19+2 and 16+3A siRNA structures.

First, each of the siRNA structures was introduced into HeLa cells using Lipofectamine 2000, and after 24 hours, total RNA was extracted from the cells by Tri-reagent kit (Ambion). 2 µg of the total RNA was ligated with 0.25 µg of GeneRacer RNA oligo without pretreatment, and the GeneRacer RNA oligo-ligated total RNA was subjected to reverse transcription using GeneRacer oligo dT and SuperScript™ III RT kit (Invitrogen). PCR was performed for 35 cycles using a GeneRacer 5' primer and a gene specific 3' primer, and then nested PCR was performed for 25 cycles using a GeneRacer 5' nested primer and a gene specific 3' nested primer. The PCR products were cloned into the T&A vector (RBC), and then sequenced.
TIG3 Gene specific 3' primer:
   5'-GGGGCAGATGGCTGTTTATTGATCC -3' (SEQ ID NO: 59)
TIG3 Gene specific 3' nested primer:
   5'-ACTTTTGCCAGCGAGAGAGGGAAAC-3' (SEQ ID NO: 60)
Lamin Gene specific 3' primer:
   5'-CCAGTGAGTCCTCCAGGTCTCGAAG-3' (SEQ ID NO: 61)
Lamin Gene specific 3' nested primer:
   5'-CCTGGCATTGTCCAGCTTGGCAGA-3' (SEQ ID NO: 62)

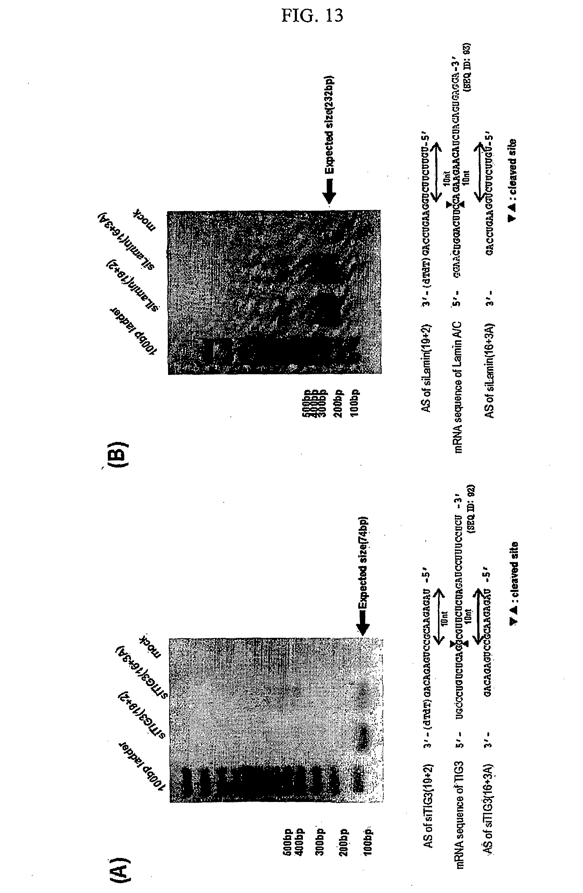

As a result, as shown in FIG. 13, TIG3 and Lamin mRNAs were all cleaved 10 nt from the 5' end of the antisense strand of each of siTIG and siLamin, and the cleavage site did not differ between the 19+2 and 16+3A siRNA structures.

These experimental results indicate that the siRNA structure according to the present invention inhibits gene expression by the same mechanism as the prior 19+2 siRNA structure.

### Example 13: Analysis of gene silencing mechanism

The siRNA structure according to the present invention contains a duplex region shorter than that of the prior 19+2 structure and has a long overhang at the end. Thus, in order to examine whether the sensitivity of the inventive siRNA structure to serum nuclease is higher than that of the 19+2 structure, the following experiment was performed.

First, 0.1 nmole of each of the 19+2 and 16+3A structures of siTIG3 was incubated in 40 µl of 10% FBS solution. 7 µl of each of the samples was taken at a predetermined point in time, and then immediately, cooled at -80°C. Then, a 3-µl fraction of each sample was separated on 15% (w/v) non-denaturing polyacrylamide gel, and then the gel was stained with EtBr and visualized by UV transillumination.

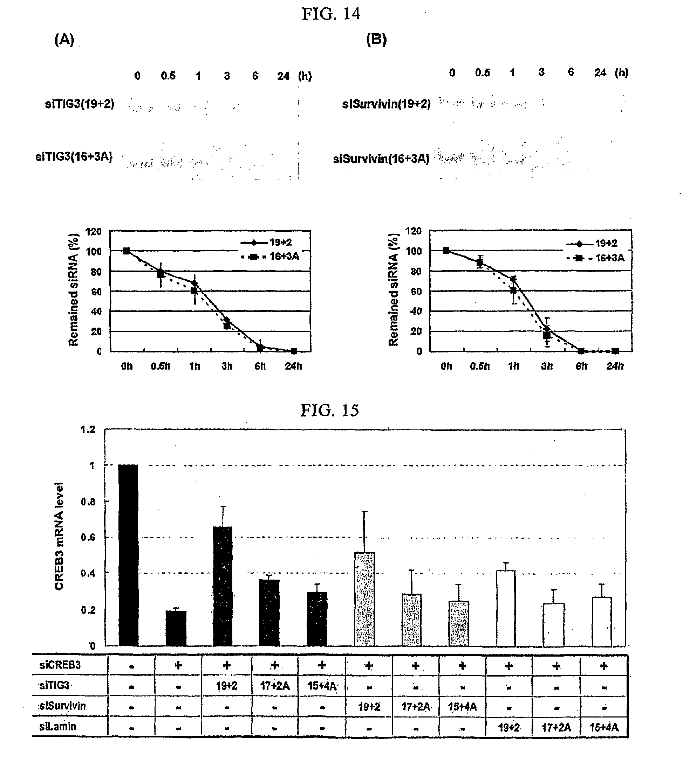

As a result, as shown in FIG. 14, the 19+2 and 16+3A siRNA structures showed similar resolution. These experimental results suggest that the stability of the siRNA structure according to the present invention is similar to that of the prior 19+2 siRNA structure.

### Example 14: Analysis I of saturation of RNAi machinery

Among the siRNAs prepared in Examples 1 to 3, that is, TIG3 mRNA-targeting siRNA (hereinafter referred to as siTIG3), Survivin mRNA-targeting siRNA (hereinafter referred to as siSurvivin) and LaminA/C mRNA-targeting siRNA (hereinafter referred to as siLamin), each of the 19+2, 17+2A and 15+4A siRNA structures was introduced into HeLa cells together with a CREB3 mRNA-targeting 19+2 siRNA structure (hereinafter referred to as siCREB3), and then the mRNA level of each gene was measured. The introduction of siRNA and the measurement of the mRNA level were performed in the same manner as in Examples 2 and 3.

### siRNA for CREB3 gene

siCREB3 antisense: 5'-GGCUCAGACUGUGUACUCC(dTdT)-3' (SEQ ID NO 63)
siCREB3 sense: 5'-GGAGUACACAGUCUGAGCC(dTdT)-3' (SEQ ID NO 64)

As a result, as shown in FIG. 15, the CREB3 mRNA level in the positive control group treated with the 19+2 structure of siCREB3 was decreased to about 20% compared to that in the negative control group introduced with no siRNA. In contrast, when the 19+2 structures of siTIG3, siSurvivin and siLamin were introduced together with the 19+2 structure of siCREB3, the CREB3 mRNA levels were reduced to 66%, 52% and 42%, respectively, compared to the mRNA level of the negative control group.

In contrast, when each of the 17+2A and 15+4A structures of siTIG3, siSurvivin and siLamin was introduced together with the 19+2 structure of siCREB3, the mRNA levels were all reduced to less than 40% compared to the mRNA level of the negative control group. Particularly, the 15+4A structures of siSurvivin and siLamin and the 17+2A structure of siLamin showed mRNA levels which were not substantially increased compared to that of the positive control group.

These experimental results indicate that, when the 19+2 siRNA structures are introduced into cells together with other siRNAs, they compete with each other, thus reducing the target gene silencing effects, but the siRNA structures according to the present invention does not substantially reduce the gene silencing effect of other siRNAs. From these results, it can be seen that the siRNA structures according to the present invention do not substantially compete with other siRNAs and do not the intracellular RNAi machinery.

### Example 15: Analysis II of saturation of RNAi machinery

In order to examine whether the siRNA structures according to the present invention interfere with intracellular miRNA (microRNA) activity, the following experiment was performed for the siTIG3 structures used in Example 7.

First, in order to evaluate the miR-21 activity of inhibiting luciferase gene expression, a luciferase reporter containing a miR-21 target sequence in the 3' untranslated region of the luciferase gene was used. When this luciferase reporter plasmid (Ambion) is introduced in HeLa cells, the luciferase activity of the cells is greatly reduced compared to that of cells introduced with a luciferase reporter control containing no miR-21 target sequence.

Thus, a pMIR-luc-based firefly luciferase reporter plasmid (Ambion) having a miR-21 binding site, a pRL-SV40 *Renilla* luciferase expression vector (Ambion) for standardization of transfection efficiency and 10 nM of siRNAs (siTIG3 structures) were introduced into Hela cells, and the standardized relative luciferase activity (the ratio of the standardized luciferase activity of the miR-21 target site-containing reporter to the standardized luciferase activity of the reporter having no miR-21 target site) was measured. The firefly luciferase activity was standardized to *Renilla* luciferase activity. The mock was introduced with the pMIR-luc-based firefly luciferase reporter plasmid and the pRL-SV40 *Renilla* luciferase expression vector without a siRNA competitor. The cells were collected and lysed in passive lysis buffer (Dual-luciferase Reproter Assay System; Promega). Then, the luciferase activity of 20 µl of each of the cell extracts was measured using the Victor3 plate reader (PerkinElmer).

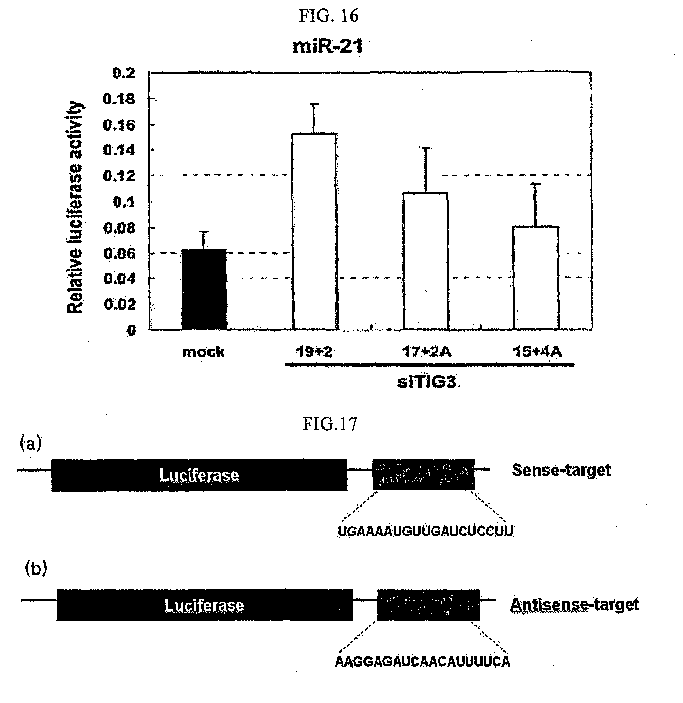

As a result, as shown in FIG. 16, the 19+2 siTIG3 structure showed a relative luciferase activity of 0.15, but the 17+2A and 15+4A siTIG3 structures showed relative luciferase activities of 0.1 and 0.08, respectively, which were lower than 0.12.

These experimental results indicate that the 19+2 siRNA structures introduced from the outside inhibit luciferase gene silencing activity mediated by miRNA, but the 17+2A and 15+4A siRNA structures show low level inhibition of luciferase gene silencing activity mediated by miRNA. Particularly, the 15+4A siRNA structure showed relative luciferase activity slightly higher than that of the mock introduced with no siRNA, suggesting that it shows low level inhibition of intracellular miRNA (microRNA) activity.

Such results together with the results of Example 14 indicate that the siRNA structures according to the present invention do not substantially saturate the intracellular RNAi machinery.

### Example 16: Analysis of off-target effects

### 16-1: Comparison between 19+2 and 16+3A siRNA structures

In order to analyze off-target effects resulting from the sense strand of the siRNA structure according to the present invention, the following experiment was performed. As used herein, the term "off-target effects" refers to any instance in which the sense strand of siRNA causes unexpected mRNA degradation or target gene silencing, even though siRNA is originally used to induce the degradation of mRNA having a sequence complementary to the antisense strand so as to obtain the effect of inhibiting the gene expression of the mRNA.

In this Example, luciferase gene-containing vectors (pMIR-REPORT™-Luciferase, Ambion) were divided into two groups. In one experimental group (A), a DNA fragment of SEQ ID NO: 65 was inserted after the luciferase gene, and in the other experimental group (B), a DNA fragment of SEQ ID NO: 66 was inserted, thus preparing DNA vectors.
SEQ ID NO 65: 5'-TGAAAATGTTGATCTCCTT
SEQ ID NO 66: 5'-AAGGAGATCAACATTTTCA

As shown in FIG. 17(a), in HeLa cells (ACTC CCL-2) introduced with the vector of experimental group A, mRNA (sense-target) containing a fragment (SEQ ID NO: 67) complementary to the sense strand of Survivin mRNA-targeting siRNA (siSurvivin) was expressed. As shown in FIG. 17(b), in HeLa cells introduced with the vector of experimental group B, mRNA (antisense-target) containing a fragment (SEQ ID NO: 68) complementary to the antisense strand of siSurvivin was expressed.
SEQ ID NO 67: 5'-UGAAAAUGUUGAUCUCCUU-3'
SEQ ID NO 68: S'-AAGGAGAUCAACAUUUUCA-3'

Thus, 200 ng of each of the vectors was introduced into HeLa cells together with the 19+2 and 16+3A siRNA structures using Lipofectamin 2000 (Invitrogen). At 24 hours after the introduction, the luciferase activity of the cells was measured by the Dual-luciferase Reporter Assay System (Promega) using the Victor3 plate reader (PerkinElmer), thus measuring the activities of the sense and antisense strands.

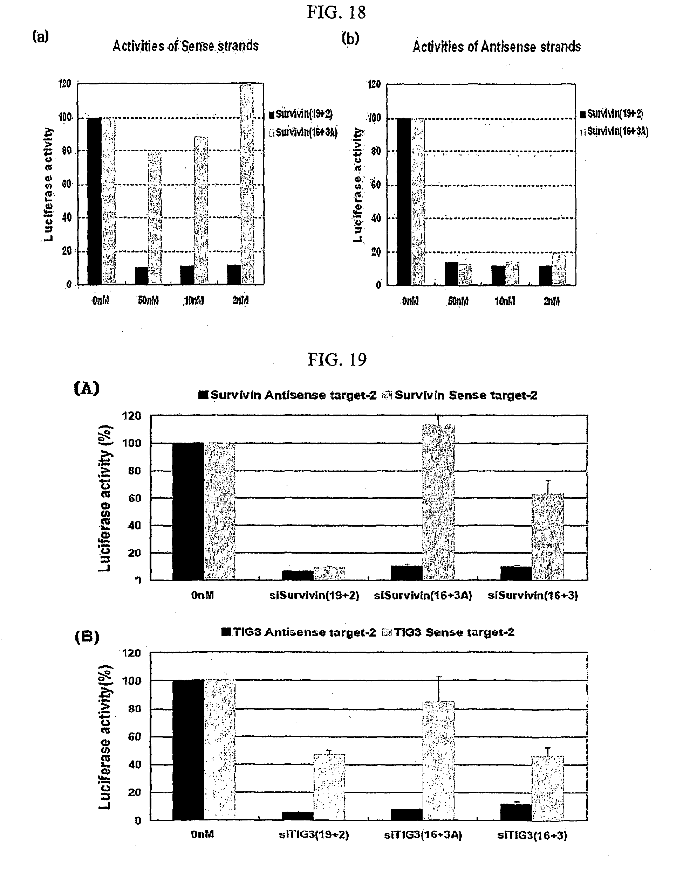

As a result, as shown in FIG. 18, the 19+2 structure of siSurvivin showed low luciferase activities in all the experimental groups A and B. On the other hand, the 16+3A structure showed low luciferase activities in the experimental group B the same as the 19+2 structure, but showed high luciferase activities in the experimental group A. This suggests that the inhibitory efficiency of luciferase activity by the antisense strand of the 16+3A structure is almost similar to that of the antisense strand of the 19+2 structure, but the activity of the sense strand of the 16+3A structure is lower than that of the sense strand of the 19+2 structure.

These experimental results suggest that the sense strand of the prior 19+2 structure causes off-target effects, but the sense strand of the 16+3A siRNA structure according to the present invention does not cause off-target effects.

### 16-2: Comparison between 16+3 structure and 16+3A structure of siRNAs

The siRNA structures according to the present invention are asymmetric, and thus the off-target effects of the sense strand thereof were analyzed comparatively with those of symmetric siRNA. For this purpose, an experiment was performed in the same manner as described above. The siRNA structures used in the experiment are shown in Tables 12 and 13 below.

**Table 12: Survivin mRNA-targeting siRNA molecules**

| | Structure | | Sequence | SEQ ID NO |
|---|---|---|---|---|
| (a) | 19+2 | antisense sense | 5'- UGAAAAUGUUGAUCUCCUU(dTdT) | 22 |
| | | | 3'-(dTdT)ACUUUUACAACUAGAGGAA | 23 |
| (b) | 16+3A | antisense sense | 5'-UGAAAAUGUUGAUCUCCUU | 24 |
| | | | 3'-ACUUUUACAAGUAGAG | 34 |
| (c) | 16+3 | antisense sense | 5'-UGAAAAUGUUGAUCUCCUU | 24 |
| | | | 3'-UAAACUUUUACAACUAGAG | 69 |

**Table 13: TIG3 mRNA-targeting siRNA molecules**

| | Structure | | Sequence | SEQ ID NO |
|---|---|---|---|---|
| (a) | 19+2 | antisense sense | 5'- UAGAGAACGCCUGAGACAG(dTdT) | 1 |
| | | | 3'- (dTdT)AUCUCUUGCGGACUCUGUC | 2 |
| (b) | 16+3A | antisense sense | 5'-UAGAGAACGCCUGAGACAG | 3 |
| | | | 3'- AUCUCUUGCGGACUCU | 8 |
| (c) | 16+3 | antisense sense | 5'-UAGAGAACGCCUGAGACAG | 3 |
| | | | 3'-UAGAUCUCUUGCGGACUCU | 70 |

As a result, as shown in FIG. 19, the off-target effects mediated by the sense strand were significantly lower in the siRNA structures of the present invention than in the symmetric 16+3 siRNA structures.

### 16-3: Comparison with siRNA, the 5' end of sense strand of which has been modified

Recent study results show that the inhibition of phosphorylation at 5' end of the sense strand of siRNA by chemical modification leads to reduced off-target effects mediated by the sense strand.

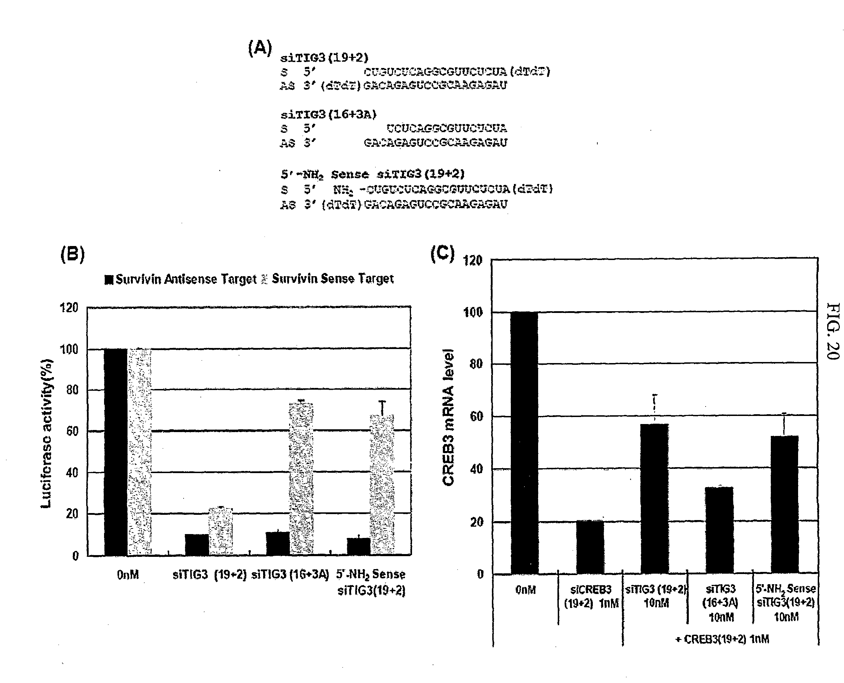

Thus, as shown in FIG. 20A, the 5' end of the 19+2 siTIG3 structure was modified with amine, and the results were analyzed. For this purpose, an experiment was performed in the same manner as described above.

As a result, as shown in FIG. 20B, the sense strand-mediated gene silencing effect of the 19+2 siRNA structure, the 5' end of the sense strand of which has been modified, was higher than that of the 16+3A siRNA structure of the present invention, but was reduced compared to the non-modified 19+2 siRNA structure. However, as shown in FIG. 20C, when it was introduced into cells together with the 19+2 structure of siCREB3, it still had potential as a strong competitor like the non-modified 19+2 structure.

In conclusion, the experimental results suggest that the siRNA structure according to the present invention is the most effective siRNA structure that dose not saturate the RNAi machinery and, at the same time, can eliminate off-target effects resulting from the sense strand.

### INDUSTRIAL APPLICABILITY

As described above, the siRNA structure according to the present invention shows excellent gene silencing efficiency without causing off-target effects by the sense strand of siRNA or interfering with other exogenous or endogenous RNAi machineries. Thus, the siRNA structure according to the present invention can substitute for prior siRNA molecules and can be advantageously used in siRNA-based gene silencing techniques such as gene therapy.
## Claims

1. A small interfering RNA molecule (siRNA molecule) comprising: a 19 nucleotide (nt) antisense strand; and a 15-17 nt sense strand having a sequence complementary to the antisense strand, wherein the 5' end of the antisense strand has a blunt end and the 3' end of the antisense strand has an overhang of 2-4 nt.

2. The siRNA molecule according to claim 1, wherein the length of the sense strand is 16 nt, and the length of the overhang is 3 nt.

3. The siRNA molecule according to claim 1, wherein the length of the sense strand is 15 nt, and the length of the overhang is 4 nt.

4. In vitro use of an siRNA molecule according to any one of claims 1 to 3 for silencing the expression of a target gene in a cell.

5. The use of claim 4, wherein the antisense strand of the siRNA molecule is complementary to the mRNA sequence of a target gene.

## Patentansprüche

1. Small-Interfering-RNA-Molekül (siRNA-Molekül), umfassend: einen 19-Nucleotid (nt)-Antisense-Strang, und einen 15-17-nt-Sense-Strang mit einer Sequenzkomplementarität mit dem Antisense-Strang, wobei das 5'-Ende des Antisense-Strangs ein stumpfes Ende hat, und das 3'-Ende des Antisense-Strangs einen Überhang von 2-4 nt hat.

2. siRNA-Molekül gemäß Anspruch 1, wobei die Länge des Sense-Strangs 16 nt ist und die Länge des Überhangs 3 nt ist.

3. siRNA-Molekül gemäß Anspruch 1, wobei die Länge des Sense-Strangs 15 nt ist und die Länge des Überhangs 4 nt ist.

4. In-vitro-Verwendung eines siRNA-Moleküls gemäß einem der Ansprüche 1 bis 3 zum Silencing der Expression eines Zielgens in einer Zelle.

5. Verwendung gemäß Anspruch 4, wobei der Antisense-Strang des siRNA-Moleküls komplementär zu der mRNA-Sequenz eines Zielgens ist.

## Revendications

1. Molécule de petit ARN interférent (molécule d'ARNsi) comprenant : un brin antisens de 19 nucléotides (nt) ; et un brin sens de 15 à 17 nt ayant une séquence complémentaire du brin antisens, dans laquelle l'extrémité 5' du brin antisens a une extrémité émoussée et l'extrémité 3' du brin antisens a une extrémité cohésive de 2 à 4 nt.

2. Molécule d'ARNsi selon la revendication 1, dans laquelle la longueur du brin sens est de 16 nt, et la longueur de l'extrémité cohésive est de 3 nt.

3. Molécule d'ARNsi selon la revendication 1, dans laquelle la longueur du brin sens est de 15 nt, et la longueur de l'extrémité cohésive est de 4 nt.

4. Utilisation in vitro d'une molécule d'ARNsi selon l'une quelconque des revendications 1 à 3, pour inhiber l'expression d'un gène cible dans une cellule.

5. Utilisation selon la revendication 4, dans laquelle le brin antisens de la molécule d'ARNsi est complémentaire de la séquence d'ARNm d'un gène cible.
